# EUROPÄISCHE PATENTSCHRIFT

(11) **EP 2 910 206 B1**
(45) Veröffentlichungstag und Bekanntmachung des Hinweises auf die Patenterteilung: **21.11.2018**
(21) Anmeldenummer: 15156467.1
(22) Anmeldetag: 25.02.2015
(51) Int. Cl.: A61B 17/17, A61B 17/70, A61B 17/34, A61B 17/86, A61B 17/88, A61B 34/20, A61B 90/00, A61B 90/98

(54) **Medizinisches Instrumentarium**
Set of medical instruments
Instrument médical

(30) Priorität: 25.02.2014 DE 102014102398
(43) Veröffentlichungstag der Anmeldung: 26.08.2015
(73) Patentinhaber: AESCULAP AG, 78532 Tuttlingen (DE)
(72) Erfinder: Wehrle, Christian, 78269 Volkertshausen (DE); Kozak, Josef, 78532 Tuttlingen (DE); Beger, Jens, 78532 Tuttlingen (DE)
(74) Vertreter: Hoeger, Stellrecht & Partner Patentanwälte mbB

(56) Entgegenhaltungen:
- US-A1- 2010 069 919
- US-A1- 2011 125 196
- US-A1- 2011 270 262
- US-A1- 2013 268 007

## Beschreibung

Die Erfindung betrifft ein medizinisches Instrumentarium, umfassend zwei oder insbesondere mehr Verankerungselemente zum Verankern an Körpergewebe und ein Stabilisierungselement, über das die zwei oder mehr Verankerungselemente verbindbar sind, sowie eine Sensoreinheit und eine Datenverarbeitungseinheit, die anhand von Sensorsignalen der Sensoreinheit die Position der Verankerungselemente relativ zueinander und/oder die Position mindestens eines Verankerungselementes relativ zum Stabilisierungselement ermittelt.

Außerdem wird ein Verfahren zum Verwenden eines medizinischen Instrumentariums beschrieben, das nicht Teil der vorliegenden Erfindung ist.

Ein medizinisches Instrumentarium der eingangs genannten Art wird beispielsweise bei der Behandlung von Wirbelfrakturen eingesetzt, bei der Verankerungselemente in Gestalt von Knochenschrauben an Wirbelkörpern fixiert und über ein Stabilisierungselement in Gestalt eines Stabes miteinander verbunden werden. Hierbei ist es bekannt, das Körpergewebe oberhalb der Wirbelkörper zu eröffnen und die Knochenschrauben unter Sicht in den Wirbelkörpern zu platzieren. In entsprechender Weise kann der Stab unter Sicht mit den Knochenschrauben fixiert werden, die zu diesem Zweck insbesondere Stabilisierungselementaufnahmen aufweisen können, in die der Stab in definierter Einführrichtung einzuführen ist. Zur Reduzierung der Invasivität schlägt die DE 10 2010 016 448 A1 vor, dass die Knochenschrauben und/oder der Stab perkutan unter Einsatz einer Ultraschallsonde lokalisiert werden. Der Operateur kann den Stab unter Ultraschallsicht sukzessive in die Stabilisierungselementaufnahmen der Knochenschrauben einfädeln.

In der US 2013/0268007 A1 ist ein Verfahren beschrieben, um mit einer Sonde perkutan selektiv unterschiedliche Abschnitte des an den Knochenschrauben angebrachten Stabes zu vermessen. Dabei wird in Bezug auf eine Referenzebene der jeweilige Winkel des Stabes ermittelt, um die Krümmung des Stabes zu bestimmen. Die Krümmung des eingesetzten Stabes wird mit einer vorgegebenen Krümmung verglichen um sicherzustellen, dass die Wirbelsäule in einer gewünschten Stellung stabilisiert wird. Dies dient zur Behandlung und Korrektur von Wirbelsäulenfehlstellungen.

Die US 2010/0069919 A1 beschreibt ein Instrumentarium mit einem Fixationssystem umfassend Knochenschrauben und einen Stab. Intrakorporal sind am Stab eine Verfolgungseinrichtung (tracking device) und an der Knochenschraube eine Zieleinrichtung (targeting device) angeordnet. Eine extrakorporale Auswerteeinheit dient dazu, die Relativposition von Stab und Knochenschraube zu ermitteln.

In der EP 2 179 703 B1 ist ein auf einem handhaltbaren Computer lauffähiges Navigationssystem beschrieben. Der Computer kann mit einem chirurgischen Instrument verbunden werden. Am Instrument ist ferner eine Markiereinrichtung angebracht, deren Bewegung im Raum von einer extern zum Computer angeordneten Navigationskamera verfolgt werden kann. Die diesbezüglichen Daten werden an den Computer übertragen und von diesem verarbeitet. Aufgabe der vorliegenden Erfindung ist es, ein medizinisches Instrumentarium bereitzustellen, das patientenfreundlicher ist.

Diese Aufgabe wird durch ein gattungsgemäßes medizinisches Instrumentarium gelöst, das gekennzeichnet ist durch eine Kopplungseinheit zum perkutanen selektiven Koppeln der Sensoreinheit mit mindestens einem Verankerungselement oder mit dem Stabilisierungselement.

Beim erfindungsgemäßen Instrumentarium kommt eine Sensoreinheit zum Einsatz, die über die Kopplungseinheit mit mindestens einem Verankerungselement oder dem Stabilisierungselement koppelbar ist. Dies erlaubt es, die Sensoreinheit in definierte Relativposition zu einem oder mehreren Verankerungselementen bzw. dem Stabilisierungselement zu bringen. Dabei ist es von Vorteil, wenn die Sensoreinheit über die Kopplungseinheit lösbar mit dem oder den Verankerungselement(en) bzw. dem Stabilisierungselement koppelbar ist. Anhand der Sensorsignale kann die Datenverarbeitungseinheit die Position der

Verankerungselemente bzw. des Stabilisierungselementes in einem Referenzkoordinatensystem der Sensoreinheit ermitteln. Möglich ist es auch, die Richtung von einem Verankerungselement zu einem weiteren Verankerungselement zu ermitteln. Dadurch ist die Möglichkeit gegeben, durch perkutane Erfassung subkutan angeordneter Verankerungselemente einen Polygonzug im Referenzkoordinatensystem zu definieren. Auch die Lage des subkutan angeordneten Stabilisierungselementes kann im Referenzkoordinatensystem definiert werden. Die Datenverarbeitungseinheit kann die Lage und Orientierung des Polygonzugs insbesondere heranziehen, wenn das Stabilisierungselement über ein Einsetzwerkzeug perkutan in den Patientenkörper eingeführt wird, um die Verankerungselemente miteinander zu verbinden. Dies ist insbesondere dann von Vorteil, wenn über die Datenverarbeitungseinheit die Lage und/oder Orientierung des Einsetzwerkzeuges und des daran angeordneten Stabilisierungselementes im Referenzkoordinatensystem verfolgt werden kann. Im Ergebnis ergibt sich eine besonders patientenfreundliche Möglichkeit, die Verankerungselemente über das Stabilisierungselement miteinander zu verbinden, wobei nur kleine Inzisionen von geringer Invasivität vorgesehen sein müssen.

Es kann insbesondere vorgesehen sein, dass die Sensoreinheit über die Kopplungseinheit sukzessive mit mindestens einem Verankerungselement koppelbar ist. Nach und nach können die Positionen der Verankerungselemente und/oder die Richtungen von zwei aufeinander folgenden Verankerungselementen im Referenzkoordinatensystem ermittelt werden. Anschließend kann beispielsweise die Lage des Stabilisierungselementes relativ zu den Verankerungselementen oder umgekehrt ermittelt werden, um diese miteinander zu verbinden. Zum Ermitteln der Richtung von einem ersten zu einem zweiten Verankerungselement ist es zum Beispiel von Vorteil, wenn die Sensoreinheit über die Kopplungseinheit mit beiden Verankerungselementen verbunden wird, worauf nachfolgend noch eingegangen wird.

Von Vorteil ist es, wenn die zwei oder mehr Verankerungselemente eine jeweilige Stabilisierungselementaufnahme aufweisen, in die das Stabilisierungselement in einer Einführrichtung einführbar ist, und wenn die Datenverarbeitungseinheit anhand der Sensorsignale der Sensoreinheit die Relativposition der Stabilisierungselementaufnahmen und die Orientierung der jeweiligen Stabilisierungselementaufnahme ermittelt, wobei die Kopplungseinheit in definierter Position und Orientierung reproduzierbar an der Stabilisierungselementaufnahme positionierbar ist. Dies erlaubt es, nicht nur die Position eines jeweiligen Verankerungselementes zu ermitteln, sondern auch die Position und insbesondere die Orientierung der jeweiligen Stabilisierungselementaufnahme. Dies ist von Vorteil um sicherzustellen, dass die Stabilisierungselementaufnahme zum einen korrekt positioniert und zum anderen so orientiert ist, dass das Stabilisierungselement in definierter Einführrichtung in die Stabilisierungselementaufnahme eingeführt werden kann.

Als vorteilhaft erweist es sich, wenn die Sensoreinheit und eine Hinweiseinheit des Instrumentariums in die Datenverarbeitungseinheit integriert sind und wenn diese tragbar ist, insbesondere in Gestalt eines Smartphones, Handheld-Computers oder Tablet-Computers. Darunter kann insbesondere verstanden werden, dass eine integrierte Einheit vorgesehen ist, die die Sensorik, die Hinweiseinheit und die Datenverarbeitung integriert. Unter "integriert" kann vorliegend insbesondere verstanden werden, dass die Sensoreinheit und die Anzeigeeinheit in einem gemeinsamen Gehäuse angeordnet sind, das auch die Datenverarbeitungseinheit umfasst. Die integrierte Einheit ist für den Operateur benutzerfreundlich handhabbar. Durch Verbinden der integrierten Einheit mit mindestens einem Verankerungselement über die Kopplungseinheit kann sich die integrierte Einheit jederzeit im Sichtfeld des Operateurs befinden, wodurch der Arbeitsablauf erheblich vereinfacht wird.

Bei der Hinweiseinheit kann es sich um eine optische Anzeigeeinheit, insbesondere mit einer Bildanzeige, handeln, und/oder um eine akustische Hinweiseinheit, die beispielsweise einen Lautsprecher umfasst.

Als vorteilhaft erweist es sich, wenn die Sensoreinheit zumindest einen der folgenden Sensoren umfasst:
- einen Positionssensor, beispielsweise einen GPS-Sensor;
- einen Neigungssensor, mit dem eine Neigung und/oder eine Änderung der Neigung der Sensoreinheit ermittelt werden kann;
- einen Kompasssensor, beispielsweise in Gestalt eines Magnetfeldsensors, insbesondere zur Ermittlung der Orientierung der Sensoreinheit relativ zum Erdmagnetfeld;
- einen Inertialsensor, insbesondere zur Ermittlung von Beschleunigungen und/oder Drehraten der Sensoreinheit;
- einen optischen Sensor, insbesondere eine Digitalkamera. Die Digitalkamera kann beispielsweise eine Markiereinrichtung erfassen, die mit einem Verankerungselement oder einem Einsetzwerkzeug für das Stabilisierungselement verbunden ist, wodurch diese im Raum verfolgt werden können. Die Digitalkamera kann auch mit einem Endoskop gekoppelt sein, mit dem subkutan die Lage des Verankerungselementes erfasst werden kann, insbesondere einer Stabilisierungselementaufnahme des Verankerungselementes.

Bei den Verankerungselementen handelt es sich beispielsweise um Knochenschrauben, insbesondere um Polyaxialschrauben mit einer jeweiligen in unterschiedlicher Orientierung ausrichtbaren Stabilisierungselementaufnahme.

Bei dem Stabilisierungselement handelt es sich beispielsweise um einen Stab, der insbesondere in definierter Einführrichtung in eine Stabilisierungselementaufnahme eines Verankerungselementes einführbar ist.

Die Kopplungseinheit umfasst beispielsweise eine Zugangstube oder bildet eine solche aus, die mit einem Verankerungselement in Gestalt einer Knochenschraube lösbar verbindbar ist, insbesondere in definierter Position und Orientierung reproduzierbar an einer Stabilisierungselementaufnahme der Knochenschraube positionierbar ist. Durch die Zugangstube kann perkutan auf das Verankerungselement eingewirkt werden, beispielsweise zur klemmenden Fixierung eines Stabes an der Knochenschraube.

Alternativ oder ergänzend kann die Kopplungseinheit ein Einsetzwerkzeug für das Stabilisierungselement umfassen oder bilden, das mit dem Stabilisierungselement lösbar verbindbar ist, insbesondere kann das Stabilisierungselement in definierter Position und Orientierung am Einsetzwerkzeug anbringbar sein. Über das Einsetzwerkzeug kann perkutan auf das Stabilisierungselement eingewirkt werden, damit die Verankerungselemente über dieses miteinander verbunden werden können.

Günstig ist es, wenn die Datenverarbeitungseinheit anhand der Positionsdaten der Verankerungselemente sowie gegebenenfalls anhand der Daten der Positionen und Orientierungen der Stabilisierungselementaufnahmen ermittelt, ob die Verankerungselemente mit dem Stabilisierungselement verbindbar sind, wobei dessen Form der Datenverarbeitungseinheit vorgebbar ist. Beispielsweise kann die Form des Stabilisierungselementes anhand von Planungsdaten der präoperativen Planung ermittelt und in der Datenverarbeitungseinheit gespeichert sein. Der Operateur kann auf diese Weise benutzerfreundlich feststellen, ob eine Verbindung der Verankerungselemente mit dem Stabilisierungselement überhaupt möglich ist.

Von Vorteil ist es, wenn das Instrumentarium eine Anzeigeeinheit umfasst und wenn die Datenverarbeitungseinheit einen Hinweis bereitstellt, mindestens ein Verankerungselement zu repositionieren, gegebenenfalls die Position und/oder die Orientierung einer Stabilisierungselementaufnahme mindestens eines Verankerungselementes zu verändern. Insbesondere bei negativer Prüfung bei der zuletzt erwähnten vorteilhaften Ausführungsform ist dadurch die Möglichkeit gegeben, dem Operateur eine Veränderung der Relativorientierung der Verankerungselemente sowie gegebenenfalls der Stabilisierungselementaufnahmen vorzuschlagen. Der Operateur kann dadurch in einer korrekten Positionierung unterstützt werden, um ein Verbinden der Verankerungselemente mit dem Stabilisierungselement zu ermöglichen.

Günstig ist es, wenn das Instrumentarium eine Mehrzahl von Stabilisierungselementen unterschiedlicher Form aufweist und wenn die Datenverarbeitungseinheit Informationen bereitstellt zur Auswahl eines zur Verbindung der Verankerungselemente geeigneten Stabilisierungselementes. Das Instrumentarium erweist sich dadurch als vielseitig und handhabungsfreundlich. Je nach Hinweis der Datenverarbeitungseinheit kann sich der Operateur eines geeigneten Stabilisierungselementes bedienen.

Von Vorteil ist es, wenn das Instrumentarium eine Stabilisierungselement-Formvorrichtung aufweist und wenn die Datenverarbeitungseinheit Informationen bereitstellt zum Formen des Stabilisierungselementes mit der Formvorrichtung durch einen Benutzer oder wenn die Datenverarbeitungseinheit die Formvorrichtung zum Formen des Stabilisierungselementes ansteuert, so dass die Verankerungselemente über das Stabilisierungselement miteinander verbindbar sind. Die Formvorrichtung kann eine ansteuerbare oder handbetätigte Formvorrichtung sein, beispielsweise eine Biegevorrichtung zum Biegen des Stabilisierungselementes, insbesondere in Gestalt eines Stabes. Anhand des Hinweises der Datenverarbeitungseinheit oder der Ansteuerung der Formvorrichtung kann dadurch sichergestellt werden, dass die Verankerungselemente über das Stabilisierungselement miteinander verbunden werden können.

Vorzugsweise umfasst das Instrumentarium eine Ultraschallsonde, die mit der Datenverarbeitungseinheit koppelbar ist, und Ultraschallbildsignale der Ultraschallsonde können von der Datenverarbeitungseinheit bevorzugt an einer Anzeigeeinheit des Instrumentariums dargestellt werden. Dies erlaubt es, über die Ultraschallsonde Körpergewebe, beispielsweise Wirbelkörper, insbesondere deren Pedikel, mit der Ultraschallsonde zu lokalisieren und an der Anzeigeeinheit zu visualisieren. Der Operateur kann dadurch beim Positionieren der Verankerungselemente unterstützt und angeleitet werden. Auch die Verankerungselemente können mit der Ultraschallsonde erfasst und bevorzugt deren Relativorientierung mit der Ultraschallsonde ermittelt werden.

Günstigerweise ist mit der Ultraschallsonde eine Markiereinrichtung gekoppelt, die von einem optischen Sensor der Sensoreinheit erfasst werden kann, so dass die Ultraschallsonde im Raum verfolgt werden kann. Ultraschallbildsignale können dadurch im Referenzkoordinatensystem ermittelt und dargestellt werden.

Vorteilhafterweise sind in der Datenverarbeitungseinheit Informationen betreffend eine vorgegebene Relativorientierung der zwei oder mehr Verankerungselemente speicherbar, und bevorzugt sind mit der Datenverarbeitungseinheit die gespeicherten Informationen mit der ermittelten Relativorientierung der zwei oder mehr Verankerungselemente vergleichbar. Eine vorgegebene Relativorientierung kann beispielsweise durch präoperative Planungsdaten bereitgestellt werden, die zum Beispiel aus Röntgen- (speziell CT-) Aufnahmen oder Ultraschallaufnahmen stammen. Durch den Vergleich der gewissermaßen gewünschten vorgegebenen Relativorientierung der Verankerungselemente mit der ermittelten tatsächlichen Relativanordnung lässt sich dadurch auf einfache Weise feststellen, ob die Verankerungselemente korrekt oder näherungsweise korrekt positioniert sind.

Die Kopplungseinheit umfasst vorzugsweise mindestens ein mit einem Verankerungselement oder mit dem Stabilisierungselement lösbar verbindbares Verlängerungselement (beispielsweise die Zugangstube bzw. das Einsetzwerkzeug) und ein Halteelement zum Haltern der Sensoreinheit am mindestens einen Verlängerungselement. Das Halteelement kann mit dem mindestens einen Verlängerungselement fest verbunden oder lösbar verbindbar sein. Das Halteelement ist im mit dem Verlängerungselement verbundenen Zustand in definierter Relativanordnung zu diesem, die im Falle einer lösbaren Verbindung reproduzierbar ist. In entsprechender Weise kann die Sensoreinheit mit dem Halteelement fest verbunden oder lösbar verbindbar sein, wobei sie in definierter Relativanordnung zu diesem ist, welche im Falle einer lösbaren Verbindung reproduzierbar ist.

Die Verbindung der Sensoreinheit mit dem Halteelement kann form- und/oder formschlüssig sein, entsprechend wie die Verbindung des Halteelementes mit dem mindestens einen Verlängerungselement sowie die Verbindung des mindestens einen Verlängerungselementes mit einem Verankerungselement bzw. dem Stabilisierungselement.

Nachfolgend wird zur vereinfachten Erläuterung der Erfindung angenommen, dass bei Vorhandensein von beispielsweise zwei Verlängerungselementen diese dieselbe Länge aufweisen, wobei sie insbesondere identisch sein können. Die vorliegende Erfindung ist jedoch nicht hierauf beschränkt, es können auch Verlängerungselemente unterschiedlicher Länge zum Einsatz kommen.

Es kann vorgesehen sein, dass zwei Verlängerungselemente über das Halteelement starr oder gelenkig miteinander verbunden oder verbindbar sind. Beispielsweise ist das Halteelement mit dem jeweiligen Verlängerungselement über ein Scharnier oder Kugelgelenk schwenkbar verbunden.

Alternativ oder ergänzend kann vorgesehen sein, dass die Länge des Halteelementes veränderbar ist. Beispielsweise ist das Halteelement teleskopierbar. Durch die Änderung der Länge des Halteelementes ist die Möglichkeit gegeben, die Verlängerungselemente parallel zueinander auszurichten, wodurch wie nachfolgend erläutert auf einfache Weise eine Richtung von einem ersten Verankerungselement zu einem zweiten Verankerungselement ermittelt werden kann.

Beispielsweise kann vorgesehen sein, dass die Richtung von einem ersten Verankerungselement zu einem zweiten Verankerungselement ermittelbar ist, indem die Kopplungseinheit mit dem ersten Verankerungselement und dem zweiten Verankerungselement über je ein Verlängerungselement verbunden ist und die Verlängerungselemente über ein Halteelement derart verbunden sind, dass die Verlängerungselemente parallel zueinander ausgerichtet sind, und dass die Sensoreinheit in vorgegebener geometrischer Beziehung zum Halteelement ausgerichtet ist. Dies erlaubt es, anhand der Orientierung der Sensoreinheit die Orientierung des Halteelementes und dadurch (aufgrund der parallelen Ausrichtung der Verlängerungselemente) die Richtung vom ersten zum zweiten Verankerungselement zu ermitteln.

Denkbar ist auch, dass die Richtung von einem ersten Verankerungselement zu einem zweiten Verankerungselement ermittelbar ist, indem die Kopplungseinheit mit dem ersten Verankerungselement und dem zweiten Verankerungselement über je ein Verlängerungselement verbunden ist und die Verlängerungselemente über ein Halteelement derart verbunden sind, dass anhand der Sensoreinheit der Winkel zwischen den Verlängerungselementen bestimmbar ist zur Ermittlung der Orientierung der dem Winkel gegenüberliegenden gedachten Verbindungslinie der Verankerungselemente miteinander. Die Verlängerungselemente können unter einem Winkel miteinander verbunden werden, wobei die Verbindung über das Halteelement erfolgen kann, an dem die Sensoreinheit gehalten ist. Dadurch wird ein Dreieck definiert mit zwei von den Verlängerungselementen definierten Seiten und einer dritten Seite, die gegebenen ist durch die gedachte Verbindungslinie der Verankerungselemente miteinander. Anhand der Sensoreinheit kann insbesondere der Winkel zwischen den Verlängerungselementen ermittelt und dadurch, speziell unter der Annahme von Verlängerungselementen gleicher Länge, die Richtung zwischen den Verankerungselementen ermittelt werden.

Es kann ferner vorgesehen sein, dass die Position eines Verankerungselementes oder des Stabilisierungselementes durch Verschwenken der Kopplungseinheit mit daran festgelegter Sensoreinheit am Verankerungselement bzw. am Stabilisierungselement ermittelbar ist, wobei die Kopplungseinheit, im Falle des Verankerungselementes, mit einer verschwenkbaren Stabilisierungselementaufnahme des Verankerungselementes gekoppelt ist. Beispielsweise wird die Kopplungseinheit mit der Sensoreinheit auf einem Kegelmantel oder einer Kugeloberfläche bewegt. Die Kegelspitze bzw. der Kugelmittelpunkt definieren dann die Position des Verankerungselementes, die im Referenzkoordinatensystem ermittelt werden kann.

Es kann ferner vorgesehen sein, dass die Richtung von einem ersten Verankerungselement zu einem zweiten Verankerungselement ermittelbar ist, indem die Kopplungseinheit synchron relativ zum ersten Verankerungselement und relativ zum zweiten Verankerungselement verschwenkt wird, wobei die Kopplungseinheit mit einer jeweiligen verschwenkbaren Stabilisierungselementaufnahme der Verankerungselemente gekoppelt ist. Beispielsweise umfasst die Kopplungseinheit zwei Verlängerungselemente, die über ein Halteelement starr miteinander verbunden sind, wobei die Verlängerungselemente parallel zueinander ausgerichtet sind. Die Sensoreinheit kann dadurch entlang eines Zylindermantels relativ zu den Verankerungselementen verschwenkt werden. Dies erlaubt es, die Richtung zwischen den Verankerungselementen zu ermitteln. Bei gelenkiger Kopplung des Halteelementes mit den Verlängerungselementen und deren paralleler Ausrichtung lässt sich die Sensoreinheit beispielsweise in der Ebene der Verlängerungselemente und des Halteelementes relativ zu den Verankerungselementen verschwenken und daraus die Richtung zwischen den Verankerungselementen ermitteln.

Es kann ferner vorgesehen sein, dass die Position eines Verankerungselementes und/oder des Stabilisierungselementes anhand von absoluten Positionsdaten (beispielsweise unter Nutzung eines GPS-Sensors) der Sensoreinheit ermittelbar ist.

Der Einsatz eines absoluten Positionssensors erlaubt es auch, absolute Positionsdaten in Positionsdaten im Referenzkoordinatensystem der Sensoreinheit zu bestimmen und umgekehrt, wodurch eine Transformation zwischen einem Welt-Koordinatensystem und dem Referenzkoordinatensystem möglich ist.

Günstig ist es, wenn das Instrumentarium eine Markiereinrichtung aufweist, die in unterschiedliche Relativpositionen zur Kopplungseinheit mit der daran angeordneten Sensoreinheit bringbar ist, wobei die Markiereinrichtung von der Sensoreinheit, insbesondere einem optischen Sensor der Sensoreinheit, erfassbar ist und von der Datenverarbeitungseinheit die Position und/oder die Orientierung der Markiereinrichtung relativ zur Sensoreinheit ermittelbar ist. Dies erlaubt es, die Markiereinrichtung bei einer Bewegung im Raum im Referenzkoordinatensystem der Sensoreinheit zu verfolgen. Positionsdaten von Gegenständen, die mit der Markiereinrichtung gekoppelt sind, können dadurch auf besonders einfache Weise ermittelt werden. Insbesondere ist es nicht erforderlich, über die Sensoreinheit hinaus eine externe Nachweisvorrichtung, wie beispielsweise eine externe Navigationskamera, vorzusehen.

Die Markiereinrichtung ist vorzugsweise mit einem Verankerungselement oder dem Stabilisierungselement koppelbar. Beispielsweise kann die Markiereinrichtung mit einem Verlängerungselement der Kopplungseinheit verbunden werden, zum Beispiel der Zugangstube bzw. dem Einsetzwerkzeug. Die Kopplung der Markiereinrichtung mit dem Verankerungselement oder dem Stabilisierungselement erfolgt in Kenntnis der Relativanordnung von Markiereinrichtung und Verankerungselement oder Stabilisierungselement. Durch Verfolgen der Markiereinrichtung kann dadurch auf die Position und/oder die Orientierung des Verankerungselementes bzw. des Stabilisierungselementes geschlossen werden.

Es kann vorgesehen sein, dass die Markiereinrichtung eine optische Anzeigeeinheit umfasst, an der die Markierelemente darstellbar sind. Beispielsweise kommt eine weitere integrierte Einheit in Gestalt eines Smartphones, Handheld-Computers oder Tablet-Computers zum Einsatz, die an einer Anzeigeeinheit Markierelemente darstellt, die von der Sensoreinheit erfasst werden können.

Alternativ kann vorgesehen sein, dass die Markiereinrichtung in einer Markierelementanordnung starr miteinander verbundene Markierelemente aufweist. Die Markierelementanordnung ist beispielsweise ein sogenannter "Rigid body".

Günstig ist es, wenn das Instrumentarium ein Einsetzwerkzeug umfasst, das mit dem Stabilisierungselement lösbar verbindbar ist und wenn die Markiereinrichtung oder die Sensoreinheit am Einsetzwerkzeug angeordnet ist oder mit diesem koppelbar ist, wobei die Position und/oder die Orientierung des Stabilisierungselementes anhand der Position und/oder der Orientierung der Markiereinrichtung relativ zur Sensoreinheit ermittelbar ist. Beispielsweise ist die Sensoreinheit über die das Einsetzwerkzeug umfassende Kopplungseinheit in definierter Relativorientierung zum Stabilisierungselement. Die Markiereinrichtung kann mit einem Verankerungselement gekoppelt und in definierter Relativorientierung zu diesem sein. Wird das Einsetzwerkzeug bewegt, kann die Lage des Stabilisierungselementes im Referenzkoordinatensystem relativ zu dem über die Markiereinrichtung referenzierten Verankerungselement ermittelt werden. Umgekehrt kann vorgesehen sein, dass die Sensoreinheit über die Kopplungseinheit mit einem Verankerungselement gekoppelt ist und dass die Markiereinrichtung mit dem Einsetzwerkzeug gekoppelt ist, wobei ebenfalls jeweils eine definierte Relativanordnung vorgesehen ist. Wie bereits erwähnt kann über die Verfolgung der Markiereinrichtung mit der Sensoreinheit die Lage des Einsetzwerkzeuges und damit die Lage des Stabilisierungselementes im Raum verfolgt werden.

Günstig ist es, wenn das Instrumentarium eine Hinweiseinheit umfasst, an der Hinweise für einen Benutzer zum Führen des Einsetzwerkzeuges ausgebbar sind, um die Verankerungselemente über das Stabilisierungselement miteinander zu verbinden. Der Benutzer kann dadurch von der Datenverarbeitungseinheit angeleitet werden, wie das Einsetzwerkzeug zu bewegen ist, damit das Stabilisierungselement mit den Verankerungselementen verbunden werden kann.

Von Vorteil ist es, wenn das Instrumentarium den Verankerungselementen zugeordnete Identifikationselemente umfasst, wobei ein jeweiliges Identifikationselement an einem Verankerungselement angeordnet oder von diesem umfasst ist und sich die Identifikationselemente voneinander unterscheiden, sowie eine Erfassungseinheit zum sukzessiven, kabellosen Erfassen der Identifikationselemente, wenn mit der Datenverarbeitungseinheit anhand von Signalen der Erfassungseinheit ermittelbar ist, ob die Reihenfolge der Erfassung der Identifikationselemente mit einer vorgegebenen oder vorgebbaren Sequenz übereinstimmt, und wenn an einer Hinweiseinheit des Instrumentariums ein diesbezüglicher positiver oder negativer Hinweis an einen Benutzer ausgebbar ist. Dies erlaubt es zum Beispiel, den Benutzer beim Einsetzen des Stabilisierungselementes zu unterstützen. So kann vorgesehen sein, dass die Verankerungselemente gemäß der vorgegebenen oder vorgebbaren Sequenz über das Stabilisierungselement miteinander zu verbinden sind. Beim Einsetzen des Stabilisierungselementes kann die Bedienperson die Identifikationselemente sukzessive mit der Erfassungseinheit erfassen. Die Datenverarbeitungseinheit kann überprüfen, ob die Reihenfolge der Identifikationselemente mit der Sequenz übereinstimmt. An der Hinweiseinheit kann dem Benutzer zur Unterstützung ein positiver oder negativer Hinweis bereitgestellt werden. Positiv ist der Hinweis, wenn das Identifikationselement mit dem gemäß der Sequenz erwarteten übereinstimmt, negativ, wenn das Identifikationselement von dem gemäß der Sequenz erwarteten abweicht. Anhand des Hinweises kann der Benutzer erkennen, ob er das korrekte Verankerungselement zum Verbinden mit dem Stabilisierungselement "ausgewählt" hat.

Das Erfassen der Identifikationselemente erfolgt vorzugsweise bei Annäherung der Erfassungseinheit an die Verankerungselemente und kann dadurch gewissermaßen "automatisch" erfolgen. Ein von der Erfassungseinheit in Richtung der Identifikationselemente ausgesandtes Erfassungssignal und/oder ein von diesen in Richtung der Erfassungseinheit ausgesandtes oder reflektiertes Signal ist/sind zu diesem Zweck bevorzugt gerichtet.

Von Vorteil ist es, wenn die Identifikationselemente RFID-Tags oder optische Identifikationselemente sind und mittels RFID-Technologie bzw. optisch von der Erfassungseinheit erfassbar sind, wobei diese als RFID-Lesegerät oder optische Erfassungseinheit ausgestaltet ist.

Es kann vorgesehen sein, dass die Sequenz der Datenverarbeitungseinheit werksseitig vorgegeben ist. Die Reihenfolge, gemäß der die Verankerungselemente zu verbinden sind, kann bereits in der Datenverarbeitungseinheit registriert sein.

Alternativ oder ergänzend kann die Sequenz der Datenverarbeitungseinheit vom Benutzer durch sukzessives Erfassen der Identifikationselemente vorgebbar sein. Der Benutzer kann die Identifikationselemente zum Beispiel vor oder nach Implantation der Verankerungselemente sukzessive erfassen und die Abfolge registrieren, längs derer das Stabilisierungselement mit diesem zu verbinden ist.

Es kann vorgesehen sein, dass die Identifikationselemente alle unmittelbar nacheinander mit der Erfassungseinheit erfasst werden können, um die Sequenz an die Datenverarbeitungseinheit zu übermitteln.

Alternativ oder ergänzend kann vorgesehen sein, dass zwischen dem Erfassen der Identifikationselemente, um der Datenverarbeitungseinheit die Sequenz vorzugeben, eine Position des jeweiligen Verankerungselementes oder Relativpositionen der Verankerungselemente zueinander auf eine der vorstehend oder nachfolgend beschriebenen Weisen bestimmt werden.

Die Hinweiseinheit kann eine optische Hinweiseinheit sein und insbesondere eine Bildanzeige aufweisen. Denkbar ist auch, dass die optische Hinweiseinheit Leuchtelemente, z.B. LED-Dioden, aufweist. Beispielsweise ist ein grünes Leuchtelement zum Anzeigen einer korrekten Reihenfolge der Erfassung und ein rotes Leuchtelement zum Anzeigen einer inkorrekten Reihenfolge der Erfassung vorgesehen.

Die Hinweiseinheit kann alternativ oder ergänzend eine akustische Hinweiseinheit sein oder umfassen, die beispielsweise einen Lautsprecher aufweist zur Ausgabe unterschiedlicher Klänge bei positivem oder negativem Hinweis.

Alternativ oder ergänzend kann vorgesehen sein, dass die Hinweiseinheit eine taktile Hinweiseinheit ist oder umfasst. Je nach positivem oder negativem Hinweis kann die Hinweiseinheit zum Beispiel unterschiedliche Vibrationen bereitstellen. Dies ist beispielsweise dann vorteilhaft, wenn die Hinweiseinheit in ein Werkzeug, zum Beispiel ein Einsetzwerkzeug für das Stabilisierungselement, integriert ist.

Die Erfassungseinheit kann getrennt von der Datenverarbeitungseinheit und der Sensoreinheit sein, ebenso wie die Hinweiseinheit.

Bei einer andersartigen Umsetzung kann vorgesehen sein, dass die Erfassungseinheit in die Datenverarbeitungseinheit oder in die Sensoreinheit integriert oder von dieser umfasst ist. Die Erfassungseinheit kann insbesondere in demselben Gehäuse mit der Datenverarbeitungseinheit oder der Sensoreinheit angeordnet sein. Die integrierte Einheit ist für den Operateur benutzerfreundlich handhabbar.

Es kann vorgesehen sein, dass die Hinweiseinheit in die Datenverarbeitungseinheit integriert oder von dieser umfasst ist. Bei der Hinweiseinheit kann es sich insbesondere um die vorstehend genannte Hinweiseinheit handeln, die mit der Sensoreinheit und der Datenverarbeitungseinheit eine integrierte Einheit bildet.

Die Erfassungseinheit kann an einem Werkzeug, insbesondere an einem Einsetzwerkzeug für das Stabilisierungselement, oder an einer Markiereinrichtung (zum Beispiel einem Rigid Body) des Instrumentariums angeordnet oder von diesem/dieser umfasst sein.

Das Instrumentarium kann einen Träger für die Erfassungseinheit und/oder die Hinweiseinheit aufweisen. Der Träger ist bevorzugt so ausgebildet, dass er am Körper eines Benutzers angeordnet und getragen werden kann, insbesondere unter einer Sterilbekleidung.

Bei einer vorteilhaften Umsetzung kann vorgesehen sein, dass das Instrumentarium ein Armband umfasst, das die Erfassungseinheit und/oder die Hinweiseinheit aufweist. Beispielsweise kann der das Armband tragende Operateur seine Hand den Verankerungselementen nähern. Deren Identifikationselemente können von der Erfassungseinheit, die in diesem Fall vorzugsweise ein RFID-Lesegerät ist, erfasst werden. Am Armband kann eine Hinweiseinheit angeordnet sein, die beispielsweise eine Bildanzeige oder Leuchtelemente umfasst.

Günstig ist es, wenn der Sensoreinheit abhängig von einem Signal der Erfassungseinheit über die Erfassung eines Identifikationselementes ein Aktivierungssignal oder ein Reset-Signal bereitstellbar ist, um die Sensoreinheit zu aktivieren oder zurückzusetzen. Dies ist zum Beispiel beim Vorgeben der Sequenz an die Datenverarbeitungseinheit wie vorstehend erläutert günstig. Wird das Identifikationselement erfasst, kann die Sensoreinheit zurückgesetzt werden, um mögliche Messfehler bei der darauffolgenden Ermittlung der Position des Verankerungselementes zu verringern. Bei einem möglichen Messfehler handelt es sich zum Beispiel um einen Drift eines Sensors, z.B. eines Inertialsensors, der zum Erfassen der Position des Verankerungselementes eingesetzt wird. Auf diese Weise kann die Genauigkeit bei der Ermittlung der Relativpositionen der Verankerungselemente gesteigert werden. Durch gezieltes Aktivieren und Deaktivieren der Sensoreinheit abhängig von einem Signal der Erfassungseinheit kann Energie zur Nutzung der Sensoreinheit gespart werden.

Zur Energieeinsparung für die Erfassungseinheit ist es von Vorteil, wenn diese mittels eines Aktivierungssignals selektiv aktivierbar und/oder deaktivierbar ist. Es kann vorgesehen sein, dass die Erfassungseinheit nur während des operativen Eingriffs aktiviert ist. Dadurch kann die Laufzeit der Erfassungseinheit, wenn diese batteriebetrieben ist, gesteigert werden. Zu Beginn des Eingriffs kann die Erfassungseinheit aktiviert und anschließend deaktiviert werden. Möglich ist auch, dass der Benutzer die Erfassungseinheit während des Eingriffs mehrfach aktiviert und/oder deaktiviert.

Das Aktivierungssignal kann der Erfassungseinheit drahtlos zuführbar sein, zum Beispiel über einen drahtlosen Einschalt- oder Abschaltimpuls. Der Impuls wird beispielsweise über ein Bluetooth Low-Energy-Funkmodul bereitgestellt.

Alternativ oder ergänzend kann vorgesehen sein, dass die Erfassungseinheit einen Beschleunigungssensor aufweist zum Bereitstellen des Aktivierungssignals abhängig von einer Bewegung der Erfassungseinheit. Durch geeignete Bewegung kann der Benutzer die Erfassungseinheit aktivieren oder deaktivieren.

Wie eingangs erwähnt betrifft die Erfindung auch ein Verfahren. Die eingangs gestellte Aufgabe wird erfindungsgemäß durch ein Verfahren gelöst, bei dem eines der vorstehend genannten Instrumentarien zum Einsatz kommt, das zwei oder insbesondere mehr Verankerungselemente zum Verankern an Körpergewebe und ein Stabilisierungselement umfasst, über das die zwei oder mehr Verankerungselemente verbindbar sind, wobei die Sensoreinheit über die Kopplungseinheit perkutan selektiv mit mindestens einem Verankerungselement oder mit dem Stabilisierungselement gekoppelt wird und mit einer Datenverarbeitungseinheit anhand von Sensorsignalen der Sensoreinheit die Position der Verankerungselemente relativ zueinander und/oder die Position mindestens eines Verankerungselementes relativ zum Stabilisierungselement ermittelt wird.

Die bereits im Zusammenhang mit der Erläuterung des erfindungsgemäßen Instrumentariums sowie vorteilhafter Ausführungsformen davon lassen sich unter Einsatz des Verfahrens ebenfalls erzielen. Diesbezüglich kann auf die voranstehenden Erläuterungen verwiesen werden.

Vorteilhafte Ausführungsbeispiele des erfindungsgemäßen Verfahrens ergeben sich durch vorteilhafte Ausführungsformen des erfindungsgemäßen Instrumentariums. Deren Merkmale können zur Definition vorteilhafter Ausführungsbeispiele des Verfahrens herangezogen werden, so dass zur Vermeidung von Wiederholungen auf voranstehende Ausführungen verwiesen wird.

Die nachfolgende Beschreibung bevorzugter Ausführungsformen der Erfindung dient im Zusammenhang mit der Zeichnung der näheren Erläuterung der Erfindung. Es zeigen:
- Figur 1:: eine schematische Darstellung eines erfindungsgemäßen Instrumentariums, mit dem ein erfindungsgemäßes Verfahren durchführbar ist;
- Figur 2:: schematisch die Verschwenkung einer Datenverarbeitungseinheit relativ zu einem Verankerungselement des Instrumentariums aus Figur 1;
- Figur 3:: schematisch die Verschwenkung der Datenverarbeitungseinheit relativ zu zwei Verankerungselementen;
- Figur 4:: schematisch die Verschwenkung der Datenverarbeitungseinheit relativ zu zwei Verankerungselementen in andersartiger Weise;
- Figur 5:: schematisch die statische Kopplung der Datenverarbeitungseinheit mit zwei Verankerungselementen;
- Figur 6:: schematisch die statische Kopplung der Datenverarbeitungseinheit mit zwei Verankerungselementen in andersartiger Weise;
- Figur 7:: schematisch die Verwendung des Instrumentariums aus Figur 1 beim Einsetzen eines Stabilisierungselementes des Instrumentariums;
- Figur 8:: schematisch die Verwendung des Instrumentariums aus Figur 1 zur Ermittlung von Positionen der Verankerungselemente; und
- Figur 9:: schematisch Verankerungselemente des Instrumentariums mit Identifikationselementen und einer Erfassungseinheit zum sukzessiven Erfassen der Identifikationselemente.

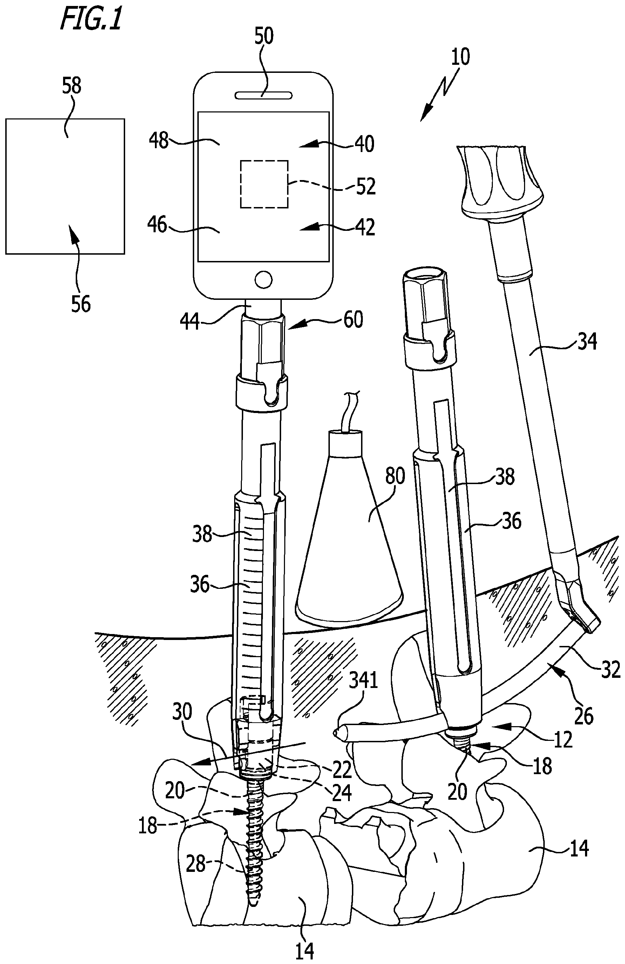

Figur 1 zeigt eine mit dem Bezugszeichen 10 belegte vorteilhafte Ausführungsform eines erfindungsgemäßen Instrumentariums. Das Instrumentarium 10 umfasst vorliegend ein chirurgisches Fixationssystem 12 zum Stabilisieren von Wirbeln 14. Zu diesem Zweck weist das Fixationssystem 12 Verankerungselemente 18 auf, vorliegend ausgestaltet als Knochenschrauben und insbesondere Polyaxialschrauben 20. Die Polyaxialschrauben 20 sind insbesondere Pedikelschrauben, die in Pedikel der Wirbel 14 eingeschraubt werden können.

Die Polyaxialschrauben umfassen Schraubenköpfe 22, die eine jeweilige Stabilisierungselementaufnahme 24 für ein Stabilisierungselement 26 des Fixationssystems 12 bilden. Die Schraubenköpfe 22 sind relativ zu Gewinden 28 der Polyaxialschrauben 20 über eine insbesondere sphärische Gelenkverbindung verschwenkbar. Bei gleichbleibender Position der Schraubenköpfe 22 wird dadurch deren Orientierung im Raum verändert. Dadurch ändert sich auch eine jeweilige Einführrichtung 30 eines Schraubenkopfes 22, in der das Stabilisierungselement 26 in den Schraubenkopf 22 eingeführt werden kann.

Das Stabilisierungselement 26 ist im vorliegenden Fall ein Stab 32, der durch die Schraubenköpfe 22 hindurchgeführt und daran in an sich bekannter Weise klemmend fixiert werden kann. Dies erlaubt es, die Polyaxialschrauben 20 über den Stab 32 miteinander zu verbinden und die Wirbel 14, 16 zu stabilisieren.

Das Fixationssystem 12 umfasst ferner ein Einsetzwerkzeug 34, an dem der Stab 32 fest ankoppelbar ist. Über das Einsetzwerkzeug 34 kann der Stab 32 perkutan bewegt werden. Die Ankopplung des Stabes 32 ist derart, dass bei bekannter Lage des Einsetzwerkzeuges 34 im Raum die Lage des Stabes 32 bekannt und festgelegt ist, insbesondere eines Stabendes 341 des Stabes 32.

Das Fixationssystem 12 umfasst ferner mindestens ein Verlängerungselement 36, vorliegend in Gestalt einer Zugangstube 38. Die Zugangstube 38 kann lösbar mit dem Schraubenkopf 22 verbunden werden, wobei die Relativanordnung des Schraubenkopfes 22 und der Zugangstube 38 bekannt und reproduzierbar ist. Dies erlaubt es, anhand der Lage der Zugangstube 38 auf die Position des Schraubenkopfes 22 und insbesondere dessen Einführöffnung zum Ermitteln der Einführrichtung 30 zu schließen. Durch die Zugangstube 38 hindurch kann perkutan auf den Schraubenkopf 22 eingewirkt werden, beispielsweise zum Verklemmen des Stabes 32.

Vorliegend ist eine Mehrzahl von Zugangstuben 38 vorgesehen, wobei jeder Polyaxialschraube 20 eine eigene Zugangstube 38 zugeordnet sein kann.

Das Instrumentarium 10 umfasst ferner eine integrierte, handhaltbare und tragbare Datenverarbeitungseinheit 40, bei der es sich beispielsweise um einen Handheld-Computer 42, ein Smartphone oder einen Tablet-Computer handeln kann. Der Computer 42 ist über ein Halteelement 44 lösbar mit einer jeweiligen Zugangstube 38 verbindbar. Dabei erfolgt die Verbindung derart, dass sowohl die Relativanordnung des Computers 42 zum Halteelement 44 als auch dessen Relativanordnung zur Zugangstube 38 bekannt und reproduzierbar ist. Dadurch kann auch eine bekannte und reproduzierbare Relativanordnung des Computers 42 zum Schraubenkopf 22 sichergestellt werden. Die Verbindung des Computers 42 über das Halteelement 44 mit der Zugangstube 38 ist kraft- und/oder formschlüssig und manuell und insbesondere werkzeuglos durchführbar. Das Halteelement 44 kann auch vom Computer 42 gelöst werden.

Die integrierte Datenverarbeitungseinheit 40 umfasst in an sich bekannter Weise einen nicht dargestellten Mikroprozessor, eine integrierte Hinweiseinheit 46 mit einer optischen Anzeigeeinheit in Gestalt eines Bildschirms 48 und mit einem Lautsprecher 50.

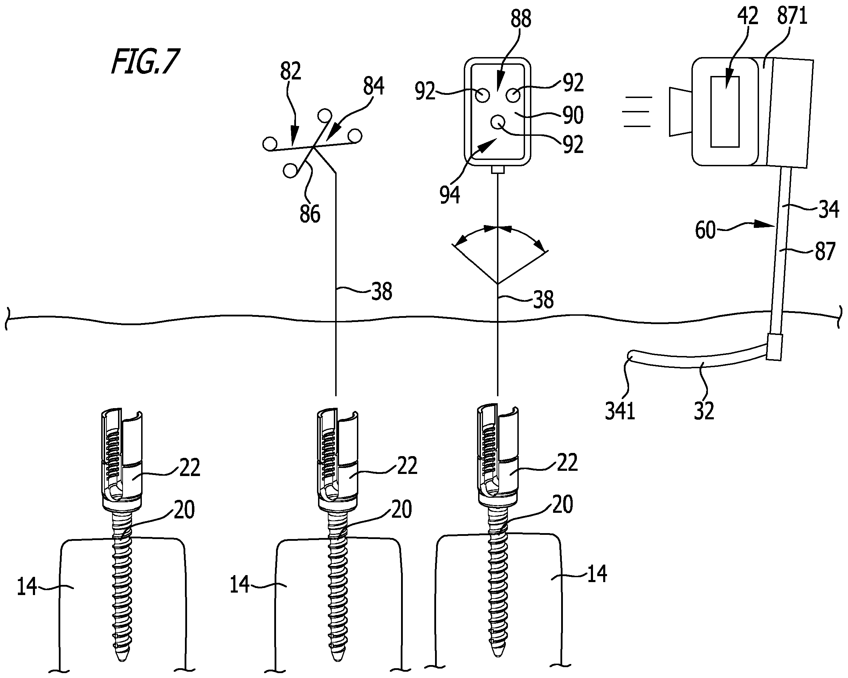

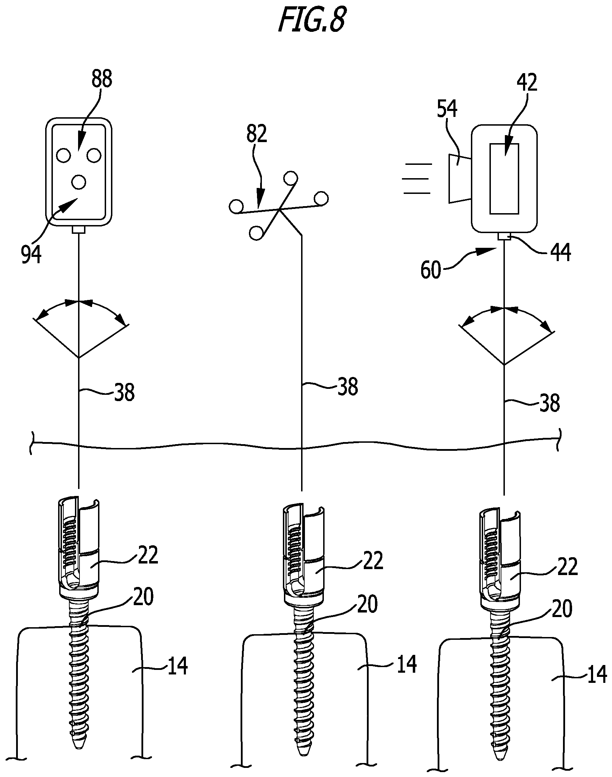

Ferner ist in die Datenverarbeitungseinheit 40 eine Sensoreinheit 52 integriert. Die Sensoreinheit 52 umfasst insbesondere eine Mehrzahl von Sensoren und stellt basierend auf deren Messungen Sensorsignale bereit, die von der Datenverarbeitungseinheit 40 ausgewertet werden können. Als Sensoren sind vorliegend vorzugsweise ein absoluter Positionssensor vorgesehen, beispielsweise ein GPS-Sensor, ein Neigungssensor zur Ermittlung einer Neigung und/oder einer Änderung der Neigung des Computers 42, ein Kompasssensor (insbesondere in Gestalt eines Magnetfeldsensors), ein Inertialsensor zur Ermittlung einer Beschleunigung und/oder Drehrate des Computers 42 sowie ein optischer Sensor. Der optische Sensor ist insbesondere ausgestaltet als Digitalkamera 54, die in den Figuren 7 und 8 schematisch dargestellt ist.

Die Sensoreinheit 52 definiert ein Referenzkoordinatensystem, in dem wie nachfolgend erläutert die Position und/oder Orientierung der Schraubenköpfe 22 und damit der Polyaxialschrauben 20 ermittelt werden können. Ferner können die Richtungen von einem Schraubenkopf 22 einer Polyaxialschraube 20 zum Schraubenkopf 22 einer weiteren Polyaxialschraube 20 ermittelt werden. Im Ergebnis kann dadurch die Relativanordnung der Schraubenköpfe 22 perkutan ermittelt werden, wodurch perkutan der Stab 32 auf operateur- und patientenfreundliche Weise durch die Schraubenköpfe 22 gefädelt und mit den Polyaxialschrauben 20 verbunden werden kann.

Das Instrumentarium 10 umfasst ferner eine Stabilisierungselement-Formvorrichtung 56 in Gestalt einer Biegevorrichtung 58. Die Biegevorrichtung 58 ist vom Computer 42 ansteuerbar, damit ein Stab in vorgegebener Form gebogen werden kann. Alternativ oder ergänzend ist die Biegevorrichtung 58 handbetätigbar. Ein Operateur kann anhand von Informationen, die der Computer 42 bereitstellt, dem Stab eine vorgegebene Form verleihen.

Die Zugangstuben 38 und das Halteelement 44 sind umfasst von einer Kopplungseinheit 60 des Instrumentariums 10. Über die Kopplungseinheit 60 kann der Computer 42 selektiv mit den Polyaxialschrauben, insbesondere deren Schraubenköpfen 22, verbunden werden. Dies erlaubt es, den Computer 42 insbesondere sukzessive mit Polyaxialschrauben 20 zu verbinden und Positions- und/oder Orientierungsdaten der Schraubenköpfe 22 selektiv und sukzessive mit dem Computer 42 zu ermitteln.

Selbstverständlich kann vorgesehen sein, dass das Fixationssystem 12 mehr als nur zwei Verankerungselemente 18 und Verlängerungselemente 36 umfasst. Über das Halteelement 44 hinaus kann die Kopplungseinheit 60 weitere und/oder andersartige Halteelemente umfassen, worauf nachfolgend noch eingegangen wird.

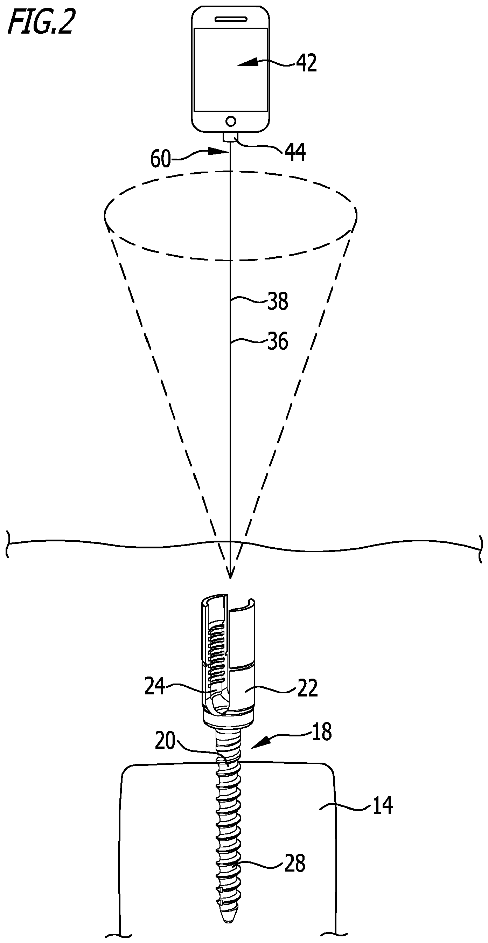

Nachfolgend wird unter Verweis insbesondere auf die Figuren 2 bis 6 beispielhaft dargestellt, wie die Position und/oder die Orientierung der Schraubenköpfe 22 und Richtungen zwischen den Schraubenköpfen 22 ermittelt werden können.

Grundsätzlich ist es denkbar, dass absolute Positionsdaten mit der Sensoreinheit 52 ermittelt werden. Aufgrund der bekannten Lageanordnung des Computers 42 zu einem jeweiligen Schraubenkopf 22 können dann absolute Positionsdaten im absoluten Koordinatensystem bestimmt werden.

In Figur 2 ist schematisch dargestellt, wie der Computer 42 durch Verschwenken der Kopplungseinheit 60, an der er angebracht ist, an der Polyaxialschraube 20 die Position des Schraubenkopfes 22 im Referenzkoordinatensystem bestimmt werden kann. Der Computer 42 wird beispielsweise auf einem Kegelmantel oder einer Kugeloberfläche verschwenkt, wodurch die Kegelspitze bzw. der Kugelmittelpunkt die Position des Schraubenkopfes 22 definieren. Auch die Einführrichtung 30 kann aufgrund der bekannten Lagebeziehung des Computers 42 zum Schraubenkopf 22 ermittelt werden.

Bei der Variante gemäß Figur 2 findet insbesondere ein Neigungssensor der Sensoreinheit 52 Verwendung. Durch einen Inertialsensor der Sensoreinheit 52 lässt sich eine Translation des Computers 42 feststellen, wodurch bei sukzessivem Verbinden des Computers 42 mit den Schraubenköpfen 22 ein Polygonzug im Referenzkoordinatensystem definiert werden kann.

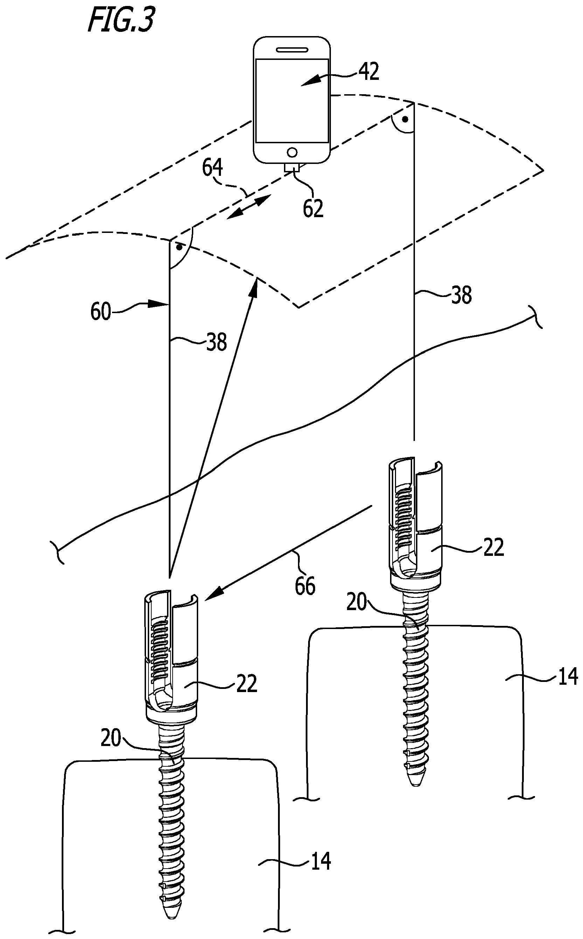

Bei der Variante gemäß Figur 3 ist ein Haltelement 62 vorgesehen, das abschnittsweise ausgestaltet ist wie das Halteelement 44, das einen Halteabschnitt des Halteelementes 62 bildet. Das Halteelement 62 umfasst ferner einen Verbindungsabschnitt 64. Der Verbindungsabschnitt 64 ist mit dem Zugangstuben 38 fest verbunden, beispielsweise an deren den Schraubenköpfen 22 abgewandten Enden und insbesondere außerhalb des Körpers des Patienten.

Bei der Variante gemäß Figur 3 wird der Computer 42 über die Kopplungseinheit 60 an den Polyaxialschrauben 20 auf einem Zylindermantel verschwenkt. Insbesondere unter Einsatz eines Neigungssensors kann dadurch auf die Orientierung des Verbindungsabschnittes 64 geschlossen werden, wodurch sich eine Richtung 66 von einer Polyaxialschraube 20 zur nächsten Polyaxialschraube 20 ergibt. Zu diesem Zweck ist es vorteilhaft, wenn der Verbindungsabschnitt 64 längenveränderlich ist, damit die Zugangstuben 38 parallel zueinander ausgerichtet werden können.

Sukzessive lassen sich nun die Richtungen von einer Polyaxialschraube 20 zur nächsten Polyaxialschraube bestimmen. Die Positionen der Schraubenköpfe 22 lassen sich beispielsweise wie vorstehend erläutert unter Einsatz eines Inertialsensors bestimmen.

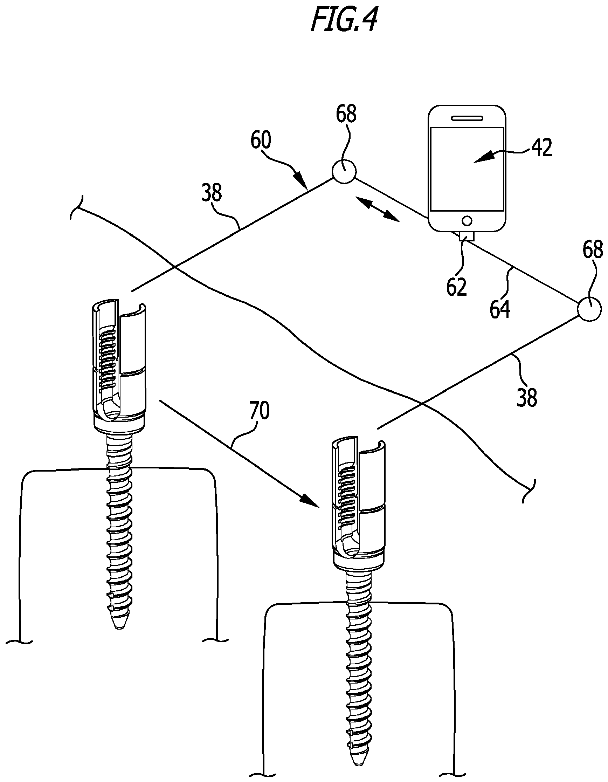

Bei der Variante gemäß Figur 4 ist der Verbindungsabschnitt 64 gelenkig mit den Zugangstuben 38 verbunden. Zu diesem Zweck sind Gelenke 68 vorgesehen, beispielsweise Scharnier- oder Kugelgelenke. Insbesondere ist eine Verschwenkung des Computers 42 in einer von den Zugangstuben 38 und dem Verbindungsabschnitt 64 definierten Ebene möglich. Bevorzugt ist auch in diesem Fall der Verbindungsabschnitt 64 längenveränderlich, um eine parallele Ausrichtung der Zugangstuben 38 zu ermöglichen.

Unter Einsatz insbesondere eines Neigungssensors und eines Inertialsensors wird die Bewegung des Computers 42 ermittelt und dadurch die Orientierung des Verbindungsabschnittes 64 und infolgedessen eine Richtung 70 von einer ersten Polyaxialschraube 20 zur nächsten Polyaxialschraube 20 ermittelt werden.

Zur weiteren Bestimmung der Lage und Orientierung der Schraubenköpfe 22 kann beispielsweise wie vorstehend erläutert vorgegangen werden.

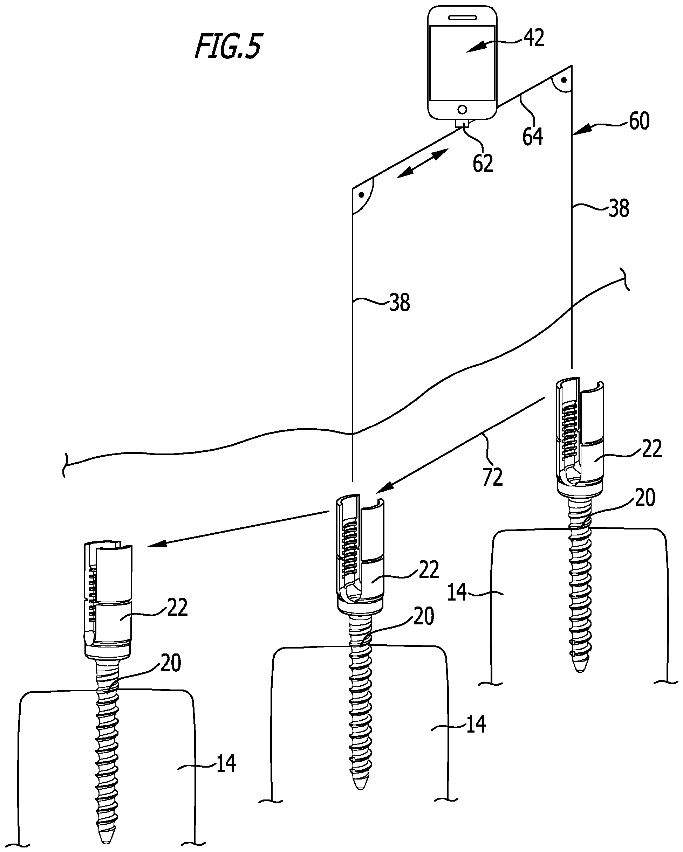

Bei der Variante gemäß Figur 5 kommt eine statische Bestimmung der Richtung 72 von zwei aufeinanderfolgenden Polyaxialschrauben 20 zum Einsatz. Dabei ist der Verbindungsabschnitt 64 wie bei der Variante gemäß Figur 3 starr mit den Zugangstuben 38 verbunden. Vorzugsweise ist der Verbindungsabschnitt 64 längenveränderlich, damit die Zugangstuben 38 parallel zueinander ausgerichtet sind.

Unter Einsatz insbesondere eines Neigungssensors und eines Kompasssensors lässt sich dadurch die Richtung 72 im Referenzkoordinatensystem bestimmen.

Zur weiteren Bestimmung von Lage und/oder Orientierung der Schraubenköpfe 22 kann wie vorstehend erläutert vorgegangen werden.

Bei den Varianten gemäß den Figuren 3 bis 5 kann vorgesehen sein, dass die Länge des Verbindungsabschnittes 64 ermittelt werden kann. Dies kann beispielsweise unter Ausnutzung eines optischen Sensors der Sensoreinheit 52 erfolgen. Durch eine Längenbestimmung des Verbindungsabschnittes 64 ist es möglich, über die Information betreffend die Richtungen 66, 70 oder 72 auch den Abstand der Polyaxialschrauben 20 im Referenzkoordinatensystem voneinander zu bestimmen.

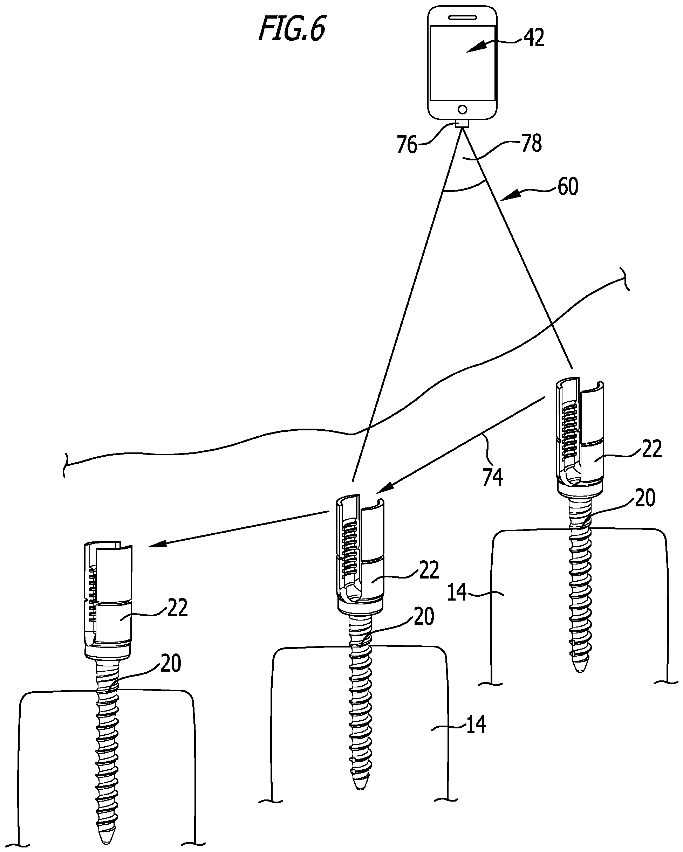

Eine weitere statische Messung einer Richtung 74 zwischen zwei Polyaxialschrauben 20 ist bei der Variante gemäß Figur 6 dargestellt. Dabei kommt ein Halteelement 76 der Kopplungseinheit 60 zum Einsatz, das dem Computer 42 mit den Zugangstuben 38 verbindet. Die Zugangstuben 38 definieren dadurch zwei Seiten eines gleichschenkligen Dreiecks, dessen dritte Seite durch eine gedachte Verbindungslinie der Schraubenköpfe 22 miteinander definiert wird. Aufgrund der bekannten Lagebeziehung des Computers 42 relativ zu den Zugangstuben 38, in die die Geometrie des Halteelementes 76 mit einfließt, ist unter Einsatz insbesondere eines Neigungssensors und eines Kompasssensors ein Winkel 78 zwischen den Zugangstuben 38 ermittelbar. Anhand des Winkels 38 kann auf die Richtung 74 geschlossen werden.

Das Halteelement 76 kann die Zugangstuben 38 auch beweglich miteinander verbinden, so dass diese unter Änderung des Winkels 78 am jeweiligen Schraubenkopf 22 verschwenkt werden können. Auch in diesem Fall ist es möglich, anhand der Daten insbesondere des Neigungs- und des Kompasssensors auf den Winkel 78 und damit die Richtung 74 zu schließen.

Zur Ermittlung der weiteren Positions- und/oder Orientierungsdaten der Schraubenköpfe 22 kann beispielsweise wie vorstehend erläutert vorgegangen werden.

Die Datenverarbeitungseinheit 40 kann den Operateur, insbesondere über den Bildschirm 48, zur Erfassung der Positions- und/oder Orientierungsdaten der Polyaxialschrauben 20, insbesondere deren Schraubenköpfe 22, anleiten. Es kann beispielsweise auf eine der anhand der Figuren 2 bis 6 erläuterten Weisen vorgegangen werden oder wie bereits erwähnt, unter Ausnutzung eines absoluten Positionssensors ein Bezug zu einem Welt-Koordinatensystem hergestellt werden.

Der Computer 42 kann anhand der ermittelten Relativanordnung der Polyaxialschrauben 20 feststellen, ob die Schraubenköpfe 22 mit dem Stab 32 überhaupt verbindbar sind. Die Form des Stabes 32 kann mit dem Computer 42 bereitgestellt und darin gespeichert werden.

Der Computer 42 kann ferner Informationen bereitstellen, anhand derer ein Benutzer einen Stab mit der Biegevorrichtung 58 in einer Form biegen kann, so dass die Polyaxialschrauben 20 miteinander verbunden werden können. Alternativ oder ergänzend kann die Biegevorrichtung 58 vom Computer 42 in geeigneter Weise angesteuert werden.

Der Computer 42 kann auch Informationen bereitstellen, aus einer Mehrzahl vorhandener Stäbe (nicht gezeigt) unterschiedlicher Form einen geeigneten Stab auszuwählen, um ein Verbinden der Polyaxialschrauben 20 zu ermöglichen.

Der Computer 42 kann Informationen bereitstellen, mindestens eine Polyaxialschraube 20 oder mindestens einen Schraubenkopf 22 zu repositionieren, damit die Polyaxialschrauben 20 über den Stab 32 miteinander verbunden werden können.

Die Relativanordnung der Polyaxialschrauben 20, wie sie anhand der Signale der Sensoreinheit 52 ermittelt wird, kann verglichen werden mit präoperativ bereitgestellten Informationen betreffend eine gewünschte Relativanordnung der Polyaxialschrauben 20 um sicherzustellen, dass das präoperative Operationsziel erreicht wird.

Das Instrumentarium 10 umfasst eine Ultraschallsonde 80, die signalwirksam mit dem Computer 42 gekoppelt werden kann. Ultraschallbildsignale können an dem Bildschirm 48 dargestellt werden. Die Ultraschallsonde 80 kann dadurch unterstützend beim Auffinden der Wirbel 14, beim Setzen der Polyaxialschrauben 20 und beim Einführen des Stabes 32 eingesetzt werden. Die Ultraschallsonde 80 kann mit einer in der Zeichnung nicht dargestellten Markiereinrichtung versehen werden, die insbesondere von einem optischen Sensor der Sensoreinheit 52 erfasst werden kann. Ultraschallbilddaten können dadurch im Referenzkoordinatensystem ermittelt und dargestellt werden.

Zum Einführen des Stabes 32 wird nachfolgend insbesondere auf Figur 7 verwiesen.

Das Instrumentarium umfasst eine Markiereinrichtung 82, die mit einer Polyaxialschraube 20 koppelbar ist. Zu diesem Zweck kann beispielsweise eine Zugangstube 38 verwendet werden, an der die Markiereinrichtung 82 in definierter Relativanordnung reproduzierbar angebracht werden kann. Alternativ kann eine eigenständige Kopplungseinheit verwendet werden.

Als Markiereinrichtung 82 kommt vorliegend beispielsweise ein sogenannter "Rigid Body" 84 zum Einsatz, der eine starre Markierelementanordnung 86 definiert.

Die Kopplungseinheit 60 umfasst das Einsetzwerkzeug 34 zum Koppeln des Computers 42 mit dem Stab 32. Das Einsetzwerkzeug 34 ist ein Verlängerungselement 87. Der Computer 42 kann über ein Halteelement 871 der Kopplungseinheit 60 in definierter Relativanordnung am Einsetzwerkzeug 34 lösbar angebracht werden. Die Relativanordnung des Stabes 32 und des Computers 42, insbesondere der Sensoreinheit 52 und des Stabendes 341, ist bekannt.

Die Relativorientierung der Markiereinrichtung 82 und des Computers 42 kann verändert werden. Beispielsweise wird die Markiereinrichtung 82 mit einer Polyaxialschraube 20 verbunden, und das Einsetzwerkzeug 34 und damit der Stab 32 werden bewegt, um den Stab 32 perkutan einzuführen. Mittels eines optischen Sensors der Sensoreinheit 52 kann die Relativbewegung der Markiereinrichtung 82 und des Computers 42 verfolgt und dadurch auf die Bewegung des jeweiligen Schraubenkopfes 22 im Referenzkoordinatensystem geschlossen werden. Dies erlaubt es, bei bekannter Relativorientierung der Schraubenköpfe 22 den Stab 32 benutzerfreundlich und patientenschonend perkutan durch die Schraubenköpfe 22 zu führen, um die Polyaxialschrauben 20 miteinander zu verbinden.

Selbstverständlich kann umgekehrt auch vorgesehen sein, dass die Markiereinrichtung 82 am Einsetzwerkzeug 34 in definierter Relativanordnung zum Stab 32 festgelegt ist und dass der Computer 42 mit einer Polyaxialschraube 20 gekoppelt ist.

Insbesondere ist die Möglichkeit gegeben, den Stab 32 sukzessive durch die Schraubenköpfe 22 hindurchzuführen, indem die Markiereinrichtung 82 sukzessive mit den Polyaxialschrauben 20 verbunden und im Raum verfolgt wird oder indem der Computer 42 sukzessive mit den Polyaxialschrauben 20 verbunden und die Markiereinrichtung 82 am Einsetzwerkzeug 34 verfolgt wird. Dies ist speziell auch möglich, ohne dass zuvor im Referenzkoordinatensystem die Lage der Schraubenköpfe 22 bestimmt wird, wie dies voranstehend beispielhaft erläutert wurde.

Es kann auch alternativ oder ergänzend zur Markiereinrichtung 82 eine Markiereinrichtung 88 vorgesehen sein mit einer optischen Anzeigeeinheit 90, an der Markierelemente 92 dargestellt sind. Beispielsweise wird die Markiereinrichtung 88 über das Halteelement 44 und eine Zugangstube 38 mit einer jeweiligen Polyaxialschraube 20 verbunden.

Es kann auch vorgesehen sein, dass die Markiereinrichtung 88 gebildet ist durch eine Datenverarbeitungseinheit 94. Die Datenverarbeitungseinheit 94 und die Datenverarbeitungseinheit 40 können insbesondere auch Positions- und Orientierungsdaten im Referenzkoordinatensystem und im Welt-Koordinatensystem übertragen, beispielsweise durch eine Funkverbindung. Zu diesem Zweck ist es möglich, dass ein jeweiliger absoluter Positionssensor zum Einsatz kommt, wodurch ein Einsatz der Digitalkamera 54 entfallen kann.

Die Markiereinrichtung 42 und/oder die Markiereinrichtung 88 können, dies ist in Figur 8 schematisch dargestellt, auch zur Bestimmung von Positions- und/oder Orientierungsdaten der Polyaxialschrauben 20, insbesondere deren Schraubenköpfen 22, eingesetzt werden. In diesem Fall ist beispielsweise vorgesehen, dass der Computer 42 über die Kopplungseinheit 60 mit einer Polyaxialschraube 20 gekoppelt ist. Die Markiereinrichtung 82 kann sukzessive mit den weiteren Polyaxialschrauben 20 gekoppelt werden. Die Relativorientierung zum Computer 42 kann insbesondere mit der Digitalkamera 54 erfasst und dadurch die Relativanordnung der Polyaxialschrauben 20 bestimmt werden. Entsprechendes gilt für die Markiereinrichtung 88, die ergänzend oder alternativ eingesetzt werden kann.

In der Zeichnung nicht dargestellt ist eine weitere Möglichkeit zur Erfassung der Lage eines Schraubenkopfes. Dabei ist die Digitalkamera 54 an ein Endoskop angekoppelt, das beispielsweise durch eine Zugangstube 38 eingeführt wird. Mit dem Endoskop kann eine charakteristische Struktur der Polyaxialschraube 20, beispielsweise des Schraubenkopfes 22, erfasst und dadurch dessen Position im Referenzkoordinatensystem ermittelt werden.

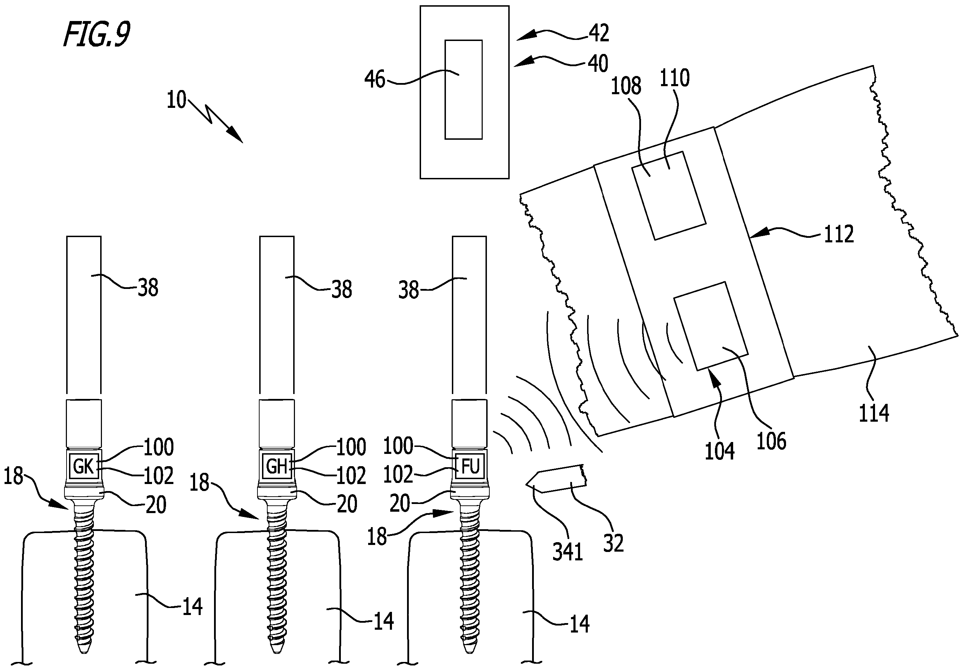

Figur 9 zeigt schematisch Wirbel 14 mit daran festgelegten Knochenschrauben 20, beispielsweise Polyaxialschrauben. Die Knochenschrauben 20 umfassen jeweils ein Identifikationselement 100. Diese sind den Knochenschrauben 20 zugeordnet und erlauben deren eindeutige Identifikation innerhalb des Instrumentariums 10.

Die Identifikationselemente 100 sind vorliegend ausgestaltet als RFID-Tags 102. Jedes RFID-Tag 102 weist eine Kennung auf, diese sind in der Zeichnung beispielhaft als "GK", "GH" und "FU" gezeigt. Die Kennung könnte auch andersartig sein.

Das Instrumentarium 10 umfasst eine Erfassungseinheit 104 zum Erfassen der Identifikationselemente 100, bei der es sich um ein RFID-Lesegerät 106 handelt. Mit dem RFID-Lesegerät 106 kann die Kennung eines jeweiligen RFID-Tags 102 erfasst werden, wenn sich dieses in der Nähe des entsprechenden RFID-Tags 102 befindet. Das RFID-Lesegerät 106 ist mit dem Computer 42 bevorzugt kabellos gekoppelt, z.B. per Funkverbindung, und kann diesem Signale bezüglich der erfassten Kennungen der RFID-Tags 102 übermitteln.

Weiter umfasst das Instrumentarium 10 eine mit dem Computer 42 gekoppelte Hinweiseinheit 108, die zum Beispiel optisch und/oder akustisch und/oder haptisch ausgebildet sein kann. Vorliegend ist die Hinweiseinheit beispielsweise als Anzeigeeinheit 110 ausgestaltet oder umfasst eine solche. Die Anzeigeeinheit 110 kann Leuchtelemente aufweisen, wie etwa Leuchtdioden, und/oder eine Bildanzeige.

Alternativ oder ergänzend zur Hinweiseinheit 108 kann die Hinweiseinheit 46 zum Einsatz kommen.

Die Erfassungseinheit könnte auch in den Computer 42 integriert sein.

Das Instrumentarium 10 kann vorzugsweise einen gemeinsamen Träger für das RFID-Lesegerät 106 und die Anzeigeeinheit 110 aufweisen, der günstigerweise am Körper des Operateurs getragen werden kann, insbesondere unter einer Sterilbekleidung. Bei der in der Zeichnung gezeigten vorteilhaften Ausführungsform ist der Träger ein Armband 112. Das Armband ist an einem schematisch dargestellten Unterarm 114 des Operateurs angelegt, zum Beispiel nahe dem Handgelenk.

Dem Computer 42 kann eine Sequenz der RFID-Tags 102 vorgegeben werden. Darunter kann insbesondere eine Information verstanden werden, in welcher Abfolge die die RFID-Tags 102 umfassenden Knochenschrauben 20 vom Operateur mit dem Stab 32 zu verbinden sind, um das Fixationssystem 12 wie gewünscht zu implantieren. Mit anderen Worten die die Sequenz der RFID-Tags 102, und damit der Knochenschrauben 20, anhand der Kennungen beim Computer 42 registriert.

Die Registrierung der Kennungen der RFID-Tags 102 im Computer 42 kann werksseitig erfolgen. Alternativ oder ergänzend kann die Registrierung beispielsweise vor dem oder bei dem Implantieren der Knochenschrauben 20 durchgeführt werden. Es kann vorgesehen sein, dass beim Ermitteln der Position und der Relativpositionen der Knochenschrauben 20 auf eine der vorstehend erläuterten Weise die Kennungen der RFID-Tags 102 registriert werden. Dabei ist es möglich, alle RFID-Tags 102 unmittelbar nacheinander zu registrieren oder abwechselnd mit der Ermittlung der Position einer der Knochenschrauben 20.

Zum Erfassen der jeweiligen Kennung kann der Operateur seinen Unterarm 114 der jeweiligen Knochenschraube 20 nähern, bis die Kennung von dessen RIFD-Tag 102 vom RFID-Lesegerät 106 erfasst und dem Computer 42 über ein Signal bereitgestellt wird. Die erfolgt gewissermaßen automatisch beim Annähern an die Knochenschraube 20.

Der Operateur kann über die Hinweiseinheiten 46 und/oder 108 zum Erfassen der Kennungen angeleitet werden.

Im vorliegenden Beispiel wird dem Computer 102 die Sequenz "FU-GH-GK" von Kennungen vorgegeben und in diesem gespeichert. In dieser Reihenfolge sind die jeweiligen Knochenschrauben 20 zu verbinden.

Beim Einsetzen des Stabes 32 ist es wünschenswert sicherzustellten, dass der Stab 32 an die richtige Knochenschraube 20 geführt wird. Hierzu können die Kennungen der RFID-Tags 102 vom Operateur sukzessive erfasst und an den Computer 42 übermittelt werden. Der Computer überprüft, ob die Reihenfolge mit der registrierten Sequenz übereinstimmt. Über einen Hinweis erhält der Operateur eine Rückmeldung, ob die korrekte Knochenschraube 20 mit dem Stab 32 "anvisiert" ist.

Beispielsweise nähert der Operateur seinen Unterarm 114 der ersten Knochenschraube 20 und erfasst über das RFID-Lesegerät 106 dessen Kennung "FU". Der Computer stellt fest, dass die erste Kennung "FU" mit dem Beginn der registrierten Sequenz übereinstimmt. An der Anzeigeeinheit 110 wird ein positiver Hinweis ausgegeben. Der Operateur führt den Stab 20 in den Schraubenkopf der ersten Knochenschraube 20 ein.

Anschließend nähert der Operateur seinen Unterarm 114 der zweiten Knochenschraube 20 und erfasst über das RFID-Lesegerät 106 dessen Kennung "GH". Der Computer stellt fest, dass die zweite Kennung "GH" mit dem Fortgang gemäß der registrierten Sequenz übereinstimmt. An der Anzeigeeinheit 110 wird ein positiver Hinweis ausgegeben (z.B. grüne Leuchtdiode). Der Operateur führt den Stab 20 in den Schraubenkopf der zweiten Knochenschraube 20 ein.

Daraufhin nähert der Operateur seinen Unterarm 114 der dritten Knochenschraube 20 und erfasst über das RFID-Lesegerät 106 dessen Kennung "GK". Der Computer stellt fest, dass die dritte Kennung "GK" mit dem weiteren Fortgang gemäß der registrierten Sequenz übereinstimmt. An der Anzeigeeinheit 110 wird ein positiver Hinweis ausgegeben. Der Operateur führt den Stab 20 in den Schraubenkopf der dritten Knochenschraube 20 ein.

Wird demgegenüber im zu vorletzt genannten Schritt vom Operateur als vermeintlich nächste Knochenschraube 20 diejenige mit der Kennung "GK" angesehen, nähert er seinen Unterarm 114 dieser (dritten) Knochenschraube 20 und erfasst über das RFID-Lesegerät 106 dessen Kennung "GK". Der Computer stellt fest, dass die Kennung "GK" mit dem Fortgang gemäß der registrierten Sequenz nicht übereinstimmt. An der Anzeigeeinheit 110 wird ein negativer Hinweis ausgegeben (z.B. rote Leuchtdiode). Der Operateur weiß dadurch, dass er eine andere Knochenschraube auswählen muss.

Dem Operateur wird auf diese Weise durch Unterstützung des Operationsablaufs die Handhabung des Instrumentariums 10 und die Implantation des Fixationssystems 12 erheblich erleichtert. Dies ist insbesondere unter Berücksichtigung einer perkutanen Implantation zu verstehen. Abweichend von der in der Zeichnung schematisch dargestellten Situation ist es in der Praxis dabei häufig nicht offensichtlich, an welcher Position sich die am nächsten mit dem Stab 32 zu verbindende Knochenschraube 20 befindet, insbesondere wenn eine Vielzahl von und/oder dicht beieinander positionierte Knochenschrauben 20 vorhanden sind.

## Patentansprüche

1. Medizinisches Instrumentarium, umfassend zwei oder insbesondere mehr Verankerungselemente (18) zum Verankern an Körpergewebe und ein Stabilisierungselement (26), über das die zwei oder mehr Verankerungselemente (18) verbindbar sind, sowie eine Sensoreinheit (52) und eine Datenverarbeitungseinheit (40), die anhand von Sensorsignalen der Sensoreinheit (52) die Position der Verankerungselemente (18) relativ zueinander und/oder die Position mindestens eines Verankerungselementes (18) relativ zum Stabilisierungselement (26) ermittelt, **gekennzeichnet durch** eine Kopplungseinheit (60) zum perkutanen selektiven Koppeln der Sensoreinheit (52) mit mindestens einem Verankerungselement (18) oder mit dem Stabilisierungselement (26).

2. Instrumentarium nach Anspruch 1, **dadurch gekennzeichnet, dass** die zwei oder mehr Verankerungselemente (18) eine jeweilige Stabilisierungselementaufnahme (24) aufweisen, in die das Stabilisierungselement (26) in einer Einführrichtung (30) einführbar ist, und dass die Datenverarbeitungseinheit anhand der Sensorsignale der Sensoreinheit (52) die Relativposition der Stabilisierungselementaufnahmen (24) und die Orientierung der jeweiligen Stabilisierungselementaufnahme (24) ermittelt, wobei die Kopplungseinheit (60) in definierter Position und Orientierung reproduzierbar an der Stabilisierungselementaufnahme (24) positionierbar ist.

3. Instrumentarium nach Anspruch 1 oder 2, **dadurch gekennzeichnet, dass** die Sensoreinheit (52) und eine Hinweiseinheit (46) des Instrumentariums in die Datenverarbeitungseinheit (40) integriert sind und dass diese tragbar ist, insbesondere in Gestalt eines Smartphones, Handheld-Computers (42) oder Tablet-Computers.

4. Instrumentarium nach einem der voranstehenden Ansprüche, **dadurch gekennzeichnet, dass** die Sensoreinheit (52) zumindest einen der folgenden Sensoren umfasst:
- einen Positionssensor;
- einen Neigungssensor;
- einen Kompasssensor;
- einen Inertialsensor;
- einen optischen Sensor, insbesondere eine Digitalkamera (54).

5. Instrumentarium nach einem der voranstehenden Ansprüche, **dadurch gekennzeichnet, dass** die Verankerungselemente (18) Knochenschrauben sind, insbesondere Polyaxialschrauben (20) mit einer jeweiligen in unterschiedlicher Orientierung ausrichtbaren Stabilisierungselementaufnahme (24), und dass das Stabilisierungselement (26) ein Stab (32) ist.

6. Instrumentarium nach einem der voranstehenden Ansprüche, **dadurch gekennzeichnet, dass** die Kopplungseinheit (60) eine Zugangstube (38) umfasst oder bildet, die mit einem Verankerungselement (18) in Gestalt einer Knochenschraube lösbar verbindbar ist, insbesondere in definierter Position und Orientierung reproduzierbar an einer Stabilisierungselementaufnahme (24) der Knochenschraube positionierbar ist, und/oder dass die Kopplungseinheit (60) ein Einsetzwerkzeug (34) umfasst oder bildet, das mit dem Stabilisierungselement (26) lösbar verbindbar ist, wobei das Stabilisierungselement (26) insbesondere in definierter Position und Orientierung am Einsetzwerkzeug anbringbar ist.

7. Instrumentarium nach einem der voranstehenden Ansprüche, **dadurch gekennzeichnet, dass** die Datenverarbeitungseinheit anhand der Positionsdaten der Verankerungselemente (18) sowie gegebenenfalls anhand der Daten der Positionen und Orientierungen der Stabilisierungselementaufnahmen (24) ermittelt, ob die Verankerungselemente (18) mit dem Stabilisierungselement (26) verbindbar sind, wobei dessen Form der Datenverarbeitungseinheit (40) vorgebbar ist.

8. Instrumentarium nach einem der voranstehenden Ansprüche, **dadurch gekennzeichnet, dass** das Instrumentarium eine Anzeigeeinheit (46) umfasst und dass die Datenverarbeitungseinheit (40) einen Hinweis bereitstellt, mindestens ein Verankerungselement (18) zu repositionieren, gegebenenfalls die Position und/oder die Orientierung einer Stabilisierungselementaufnahme (24) mindestens eines Verankerungselementes (18) zu verändern.

9. Instrumentarium nach einem der voranstehenden Ansprüche, **dadurch gekennzeichnet, dass** das Instrumentarium (10) eine Mehrzahl von Stabilisierungselementen (26) unterschiedlicher Form aufweist und dass die Datenverarbeitungseinheit (40) Informationen bereitstellt zur Auswahl eines zur Verbindung der Verankerungselemente (18) geeigneten Stabilisierungselementes (26), und/oder dass das Instrumentarium (10) eine Stabilisierungselement-Formvorrichtung (56) aufweist und dass die Datenverarbeitungseinheit (40) Informationen bereitstellt zum Formen des Stabilisierungselementes (26) mit der Formvorrichtung (56) durch einen Benutzer oder dass die Datenverarbeitungseinheit die Formvorrichtung (56) zum Formen des Stabilisierungselementes (26) ansteuert, so dass die Verankerungselemente (18) über das Stabilisierungselement (26) miteinander verbindbar sind.

10. Instrumentarium nach einem der voranstehenden Ansprüche, **dadurch gekennzeichnet, dass** die Kopplungseinheit (60) mindestens ein mit einem Verankerungselement (18) oder mit dem Stabilisierungselement (26) lösbar verbindbares Verlängerungselement (36) umfasst und ein Halteelement (44, 62, 76) zum Haltern der Sensoreinheit (52) am mindestens einen Verlängerungselement (36).

11. Instrumentarium nach einem der voranstehenden Ansprüche, **dadurch gekennzeichnet, dass** die Position eines Verankerungselementes (18) und/oder des Stabilisierungselementes (26) anhand absoluter Positionsdaten der Sensoreinheit (52) ermittelbar ist.

12. Instrumentarium nach einem der voranstehenden Ansprüche, **dadurch gekennzeichnet, dass** das Instrumentarium (10) eine Markiereinrichtung (82, 88) aufweist, die in unterschiedliche Relativpositionen zur Kopplungseinheit (60) mit der daran angeordneten Sensoreinheit (52) bringbar ist, wobei die Markiereinrichtung (82, 88) von der Sensoreinheit (52), insbesondere einem optischen Sensor der Sensoreinheit (52), erfassbar ist und von der Datenverarbeitungseinheit (40) die Position und/oder die Orientierung der Markiereinrichtung (82, 88) relativ zur Sensoreinheit (52) ermittelbar ist.

13. Instrumentarium nach Anspruch 12, **dadurch gekennzeichnet, dass** die Markiereinrichtung (82, 88) mit einem Verankerungselement (18) oder mit dem Stabilisierungselement (26) koppelbar ist.

14. Instrumentarium nach Anspruch 12 oder 13, **dadurch gekennzeichnet, dass** das Instrumentarium ein Einsetzwerkzeug (34) umfasst, das mit dem Stabilisierungselement (26) lösbar verbindbar ist und dass die Markiereinrichtung (82, 88) oder die Sensoreinheit (52) am Einsetzwerkzeug (34) angeordnet oder mit diesem koppelbar ist, wobei die Position und/oder die Orientierung des Stabilisierungselementes (26) anhand der Position und/oder der Orientierung der Markiereinrichtung (82, 88) relativ zur Sensoreinheit (52) ermittelbar ist, und/oder dass das Instrumentarium (10) eine Hinweiseinheit (46) umfasst, an der Hinweise für einen Benutzer zum Führen des Einsetzwerkzeuges (34) ausgebbar sind, um die Verankerungselemente (18) über das Stabilisierungselement (26) miteinander zu verbinden.

15. Instrumentarium nach einem der voranstehenden Ansprüche, **dadurch gekennzeichnet, dass** das Instrumentarium (10) den Verankerungselementen (18) zugeordnete Identifikationselemente (100) umfasst, wobei ein jeweiliges Identifikationselement (100) an einem Verankerungselement (18) angeordnet oder von diesem umfasst ist und sich die Identifikationselemente (100) voneinander unterscheiden, sowie eine Erfassungseinheit (104) zum sukzessiven, kabellosen Erfassen der Identifikationselemente (100), dass mit der Datenverarbeitungseinheit (40) anhand von Signalen der Erfassungseinheit (104) ermittelbar ist, ob die Reihenfolge der Erfassung der Identifikationselemente (100) mit einer vorgegebenen oder vorgebbaren Sequenz übereinstimmt, und dass an einer Hinweiseinheit (46, 108) des Instrumentariums (10) ein diesbezüglicher positiver oder negativer Hinweis an einen Benutzer ausgebbar ist.

## Claims

1. Medical instrumentation, comprising two or, in particular, more anchoring elements (18) for anchoring on body tissue and a stabilization element (26) via which the two or more anchoring elements (18) are connectable, as well as a sensor unit (52) and a data processing unit (40) which determines from sensor signals of the sensor unit (52) the position of the anchoring elements (18) relative to one another and/or the position of at least on anchoring element (18) relative to the stabilization element (26), **characterized by** a coupling unit (60) for percutaneous selective coupling of the sensor unit (52) to at least one anchoring element (18) or to the stabilization element (26).

2. Instrumentation in accordance with claim 1, **characterized in that** the two or more anchoring elements (18) comprise a respective stabilization element receptacle (24) into which the stabilization element (26) is insertable in a direction of insertion (30), and **in that** the data processing unit determines from the sensor signals of the sensor unit (52) the relative position of the stabilization element receptacles (24) and the orientation of the respective stabilization element receptacle (24), with the coupling unit (60) being positionable in a defined position and orientation in a reproducible manner at the stabilization element receptacle (24).

3. Instrumentation in accordance with claim 1 or 2, **characterized in that** the sensor unit (52) and an indication unit (46) of the instrumentation are integrated in the data processing unit (40), and **in that** this is portable, in particular, in the form of a smartphone, handheld computer (42) or tablet computer.

4. Instrumentation in accordance with any one of the preceding claims, **characterized in that** the sensor unit (52) comprises at least one of the following sensors:
- a position sensor;
- an inclination sensor;
- a compass sensor;
- an inertial sensor;
- an optical sensor, in particular, a digital camera (54).

5. Instrumentation in accordance with any one of the preceding claims, **characterized in that** the anchoring elements (18) are bone screws, in particular, polyaxial screws (20) with a respective stabilization element receptacle (24) alignable in different orientations, and **in that** the stabilization element (26) is a rod (32).

6. Instrumentation in accordance with any one of the preceding claims, **characterized in that** the coupling unit (60) comprises or forms an access tube (38) which is releasably connectable to an anchoring element (18) in the form of a bone screw, in particular, is positionable in a defined position and orientation in a reproducible manner at a stabilization element receptacle (24) of the bone screw, and/or **in that** the coupling unit (60) comprises or forms an insertion tool (34) which is releasably connectable to the stabilization element (26), the stabilization element (26) being attachable, in particular, in a defined position and orientation to the insertion tool.

7. Instrumentation in accordance with any one of the preceding claims, **characterized in that** the data processing unit determines from the position data of the anchoring elements (18) and, where appropriate, from the data of the positions and orientations of the stabilization element receptacles (24) whether the anchoring elements (18) are connectable via the stabilization element (26), it being possible for the shape of the stabilization element (26) to be preset in the data processing unit (40).

8. Instrumentation in accordance with any one of the preceding claims, **characterized in that** the instrumentation comprises a display unit (46), and **in that** the data processing unit (40) provides an indication to reposition at least one anchoring element (18), where appropriate, to change the position and/or the orientation of a stabilization element receptacle (24) of at least one anchoring element (18).

9. Instrumentation in accordance with any one of the preceding claims, **characterized in that** the instrumentation (10) comprises a plurality of stabilization elements (26) of different shapes, and **in that** the data processing unit (40) provides information for selection of a stabilization element (26) suitable for connection of the anchoring elements (18), and/or **in that** the instrumentation (10) comprises a stabilization element shaping device (56), and **in that** the data processing unit (40) provides information for the shaping of the stabilization element (26) with the shaping device (56) by a user, or that the data processing unit controls the shaping device (56) for shaping the stabilization element (26), so that the anchoring elements (18) are connectable to one another via the stabilization element (26).

10. Instrumentation in accordance with any one of the preceding claims, **characterized in that** the coupling unit (60) comprises at least one extension element (36) releasably connectable to an anchoring element (18) or to the stabilization element (26) and a retaining element (44, 62, 76) for retaining the sensor unit (52) on the at least one extension element (36).

11. Instrumentation in accordance with any one of the preceding claims, **characterized in that** the position of an anchoring element (18) and/or of the stabilization element (26) is determinable from absolute position data of the sensor unit (52).

12. Instrumentation in accordance with any one of the preceding claims, **characterized in that** the instrumentation (10) comprises a marking device (82, 88) which is movable into different relative positions in relation to the coupling unit (60) with the sensor unit (52) arranged thereon, the marking device (82, 88) being detectable by the sensor unit (52), in particular, by an optical sensor of the sensor unit (52), and the position and/or the orientation of the marking device (82, 88) relative to the sensor unit (52) being determinable by the data processing unit (40).

13. Instrumentation in accordance with claim 12, **characterized in that** the marking device (82, 88) can be coupled to an anchoring element (18) or to the stabilization element (26).

14. Instrumentation in accordance with claim 12 or 13, **characterized in that** the instrumentation comprises an insertion tool (34) which is releasably connectable to the stabilization element (26), and **in that** the marking device (82, 88) or the sensor unit (52) is arranged on or can be coupled to the insertion tool (34), the position and/or the orientation of the stabilization element (26) being determinable from the position and/or the orientation of the marking device (82, 88) relative to the sensor unit (52), and/or **in that** the instrumentation (10) comprises an indication unit (46) on which indications are displayable for a user for guiding the insertion tool (34), in order to connect the anchoring elements (18) to one another via the stabilization element (26).

15. Instrumentation in accordance with any one of the preceding claims, **characterized in that** the instrumentation (10) comprises identification elements (100) allocated to the anchoring elements (18), a respective identification element (100) being arranged on or included in an anchoring element (18), and the identification elements (100) differing from one another, and a detection unit (104) for successive, cable-free detection of the identification elements (100), that it is determinable by the data processing unit (40) from signals of the detection unit (104) whether the order in which the identification elements (100) are detected matches a preset or presettable sequence, and a positive or negative indication relating to this being displayable to a user on an indication unit (46, 108) of the instrumentation (10).

## Revendications

1. Instrumentation médicale, comprenant deux ou en particulier plusieurs éléments d'ancrage (18) pour l'ancrage aux tissus corporels et un élément de stabilisation (26) par-dessus lequel les deux ou plusieurs éléments d'ancrage (18) peuvent être raccordés, ainsi qu'une unité de capteur (52) et une unité de traitement de données (40) qui à partir de signaux de capteurs de l'unité de capteur (52) détermine la position des éléments d'ancrage (18) entre eux et/ou la position d'au moins un élément d'ancrage (18) par rapport à l'élément de stabilisation (26), **caractérisé par** une unité de couplage (60) pour le couplage sélectif percutané de l'unité de capteur (52) avec au moins un élément d'ancrage (18) ou avec l'élément de stabilisation (26).

2. Instrumentation selon la revendication 1, **caractérisée en ce que** les deux ou plusieurs éléments d'ancrage (18) présentent un logement d'élément de stabilisation (24) respectif dans lequel l'élément de stabilisation (26) peut être inséré dans une direction d'insertion (30), et **en ce que** l'unité de traitement de données détermine la position relative des logements d'élément de stabilisation (24) et l'orientation du logement d'élément de stabilisation (24) respectif à partir des signaux de capteurs de l'unité de capteur (52), dans laquelle l'unité de couplage (60) est positionnable contre le logement d'élément de stabilisation (24) de façon reproductible dans une position et une orientation définies.

3. Instrumentation selon la revendication 1 ou 2, **caractérisée en ce que** l'unité de capteur (52) et une unité de renseignement (46) de l'instrumentation sont intégrées dans l'unité de traitement de données (40) et **en ce que** celle-ci peut être portée, en particulier sous la forme d'un téléphone intelligent, ordinateur de poche (42) ou tablette numérique.

4. Instrumentation selon l'une des revendications précédentes, **caractérisée en ce que** l'unité de capteur (52) comprend au moins l'un des capteurs suivants :
- un capteur de position ;
- un capteur d'inclinaison ;
- un capteur de boussole ;
- un capteur inertiel ;
- un capteur optique, en particulier une caméra numérique (54).

5. Instrumentation selon l'une des revendications précédentes, **caractérisée en ce que** les éléments d'ancrage (18) sont des vis à os, en particulier des vis polyaxiales (20) avec un logement d'élément de stabilisation (24) respectif pouvant être dirigé dans une orientation différente, et **en ce que** l'élément de stabilisation (26) est un bâton (32).

6. Instrumentation selon l'une des revendications précédentes, **caractérisée en ce que** l'unité de couplage (60) comprend ou constitue un tube d'accès (38) qui peut être raccordée de façon amovible à un élément d'ancrage (18) sous la forme d'une vis à os, en particulier est positionnable contre un logement d'élément de stabilisation (24) de façon reproductible dans une position et une orientation définies, et/ou **en ce que** l'unité de couplage (60) comprend ou constitue un outil de montage (34) qui peut être raccordé de façon amovible à l'élément de stabilisation (26), dans laquelle l'élément de stabilisation (26) peut être fixé à l'outil de montage en particulier dans une position et une orientation définies.

7. Instrumentation selon l'une des revendications précédentes, **caractérisée en ce que** l'unité de traitement de données détermine, à partir des données de position des éléments d'ancrage (18) ainsi que le cas échéant à partir des données des positions et orientations des logements d'élément de stabilisation (24), si les éléments d'ancrage (18) peuvent être raccordés au moyen de l'élément de stabilisation (26), dans laquelle la forme de celui-ci peut être prescrite auprès de l'unité de traitement de données (40).

8. Instrumentation selon l'une des revendications précédentes, **caractérisée en ce que** l'instrumentation comprend une unité de signalement (46) et **en ce que** l'unité de traitement de données (40) fournit un renseignement indiquant de repositionner au moins un élément d'ancrage (18), le cas échéant de modifier la position et/ou l'orientation d'un logement d'élément de stabilisation (24) d'au moins un élément d'ancrage (18).

9. Instrumentation selon l'une des revendications précédentes, **caractérisée en ce que** l'instrumentation (10) présente une pluralité d'éléments de stabilisation (26) de différentes formes et **en ce que** l'unité de traitement de données (40) fournit des informations pour le choix d'un élément de stabilisation (26) approprié pour raccorder les éléments d'ancrage (18), et/ou **en ce que** l'instrumentation (10) présente un dispositif de mise en forme d'élément de stabilisation (56) et **en ce que** l'unité de traitement de données (40) fournit des informations pour la mise en forme de l'élément de stabilisation (26) avec le dispositif de mise en forme (56) par un utilisateur ou **en ce que** l'unité de traitement de données commande le dispositif de mise en forme (56) pour la mise en forme de l'élément de stabilisation (26), de sorte que les éléments d'ancrage (18) puissent être raccordés entre eux par-dessus l'élément de stabilisation (26).

10. Instrumentation selon l'une des revendications précédentes, **caractérisée en ce que** l'unité de couplage (60) comprend au moins un élément d'allongement (36) pouvant être raccordé de façon amovible à un élément d'ancrage (18) ou à l'élément de stabilisation (26) et un élément de maintien (44, 62, 76) pour maintenir l'unité de capteur (52) contre le au moins un élément d'allongement (36).

11. Instrumentation selon l'une des revendications précédentes, **caractérisée en ce que** la position d'un élément d'ancrage (18) et/ou de l'élément de stabilisation (26) peut être déterminée à partir de données de position absolues de l'unité de capteur (52).

12. Instrumentation selon l'une des revendications précédentes, **caractérisée en ce que** l'instrumentation (10) présente un dispositif de marquage (82, 88) qui peut être amené dans différentes positions relatives par rapport à l'unité de couplage (60), avec l'unité de capteur (52) agencée contre celle-ci, dans laquelle le dispositif de marquage (82, 88) est détectable par l'unité de capteur (52), en particulier par un capteur optique de l'unité de capteur (52), et la position et/ou l'orientation du dispositif de marquage (82, 88) peut être déterminée par rapport à l'unité de capteur (52) au moyen de l'unité de traitement de données (40).

13. Instrumentation selon la revendication 12, **caractérisée en ce que** le dispositif de marquage (82, 88) peut être couplé à un élément d'ancrage (18) ou à l'élément de stabilisation (26).

14. Instrumentation selon la revendication 12 ou 13, **caractérisée en ce que** l'instrumentation comprend un outil de montage (34) qui peut être raccordé de façon amovible à l'élément de stabilisation (26) et **en ce que** le dispositif de marquage (82, 88) ou l'unité de capteur (52) est agencé(e) contre l'outil de montage (34) ou peut être couplé(e) à celui-ci, dans laquelle la position et/ou l'orientation de l'élément de stabilisation (26) peut être déterminée à partir de la position et/ou de l'orientation du dispositif de marquage (82, 88) par rapport à l'unité de capteur (52), et/ou **en ce que** l'instrumentation (10) comprend une unité de renseignement (46) à laquelle des renseignements peuvent être délivrés pour un utilisateur en vue de guider l'outil de montage (34) afin de raccorder entre eux les éléments d'ancrage (18) au moyen de l'élément de stabilisation (26).

15. Instrumentation selon l'une des revendications précédentes, **caractérisée en ce que** l'instrumentation (10) comprend des éléments d'identification (100) affectés aux éléments d'ancrage (18), dans laquelle un élément d'identification (100) respectif est agencé contre un élément d'ancrage (18) ou y est compris et les éléments d'identification (100) se différencient entre eux, ainsi qu'une unité de détection (104) pour une détection successive et sans fil des éléments d'identification (100), **en ce qu'**avec l'unité de traitement de données (40) il est possible de déterminer à partir de signaux de l'unité de détection (104) si l'ordre de la détection des éléments d'identification (100) concorde avec une séquence prédéfinie ou pouvant être prescrite, et **en ce qu'**au niveau d'une unité de renseignement (46, 108) de l'instrumentation (10) un renseignement positif ou négatif à cet égard peut être délivré à un utilisateur.
